# EUROPEAN PATENT APPLICATION

(11) **EP 3 666 773 A1**
(43) Date of publication of application: **17.06.2020**
(21) Application number: 18211726.7
(22) Date of filing: 11.12.2018
(51) Int. Cl.: C07D 471/04

(54) **PROCESS FOR PREPARING APIXABAN**

(71) Applicant: KRKA, D.D., Novo Mesto, 8501 Novo mesto (SI)
(72) Inventor: KASTELIC, Andreja Radman, 47276 Zakanje (HR); KRALJ, David, 8310 Sentjernej (SI); JUG, Katja, 8350 Dolenjske Toplice (SI); BENKIC, Primoz, 1000 Ljubljana (SI); BÖHM, Jerneja, 8250 Brezice (SI); KOLENC, Ivanka, 8000 Novo mesto (SI)
(74) Representative: Hoefer & Partner Patentanwälte mbB

(57) **Abstract**

The present invention relates to process for preparing apixaban, in particular polymorphic form N-1 thereof, as well as to a method for the preparation of crystalline apixaban, especially apixaban polymorphic form N-1.

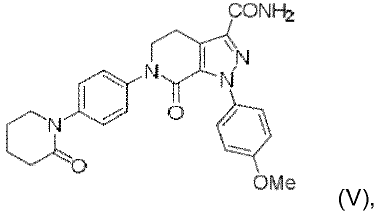

## Description

The present invention relates to a process for preparing apixaban, in particular polymorphic form N-1 thereof, as well as to a method for the preparation of crystalline apixaban, especially apixaban polymorphic form N-1.

### Background of the invention

Apixaban is a compound having the chemical name 1-(4-methoxyphenyl)-7-oxo-6-[4-(2-oxo-1-piperidinyl)phenyl]-4,5,6,7-tetrahydro-1H-pyrazolo[3,4-c]pyridine-3-carboxamide, and has the structure shown below in Formula (V):

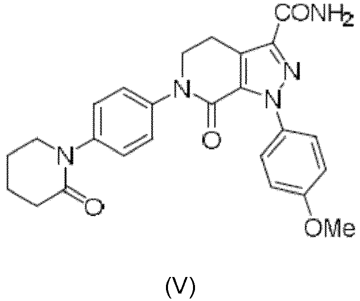

Apixaban is a factor Xa inhibitor and can be used for the treatment or prevention of a thromboembolic disorder. Apixaban is disclosed in U.S. Patent No. 6,967,208. Apixaban has been described as a poorly soluble compound.

Approaches for preparing 4,5-dihydro-pyrazolo[3,4-c]pyrid-2-ones are known in the art. For example, WO 2007/001385 A2 discloses procedures for preparing 4,5-dihydro-pyrazolo[3,4-c]pyrid-2-ones. Furthermore, WO 2012/168364 A1 describes a synthesis providing apixaban, and WO 2016/035007A2 discloses a process for the preparation of apixaban and intermediates thereof. Moreover, Brad D. Maxwell et al. describe syntheses and in vitro biotransformation studies of [14C]apixaban in Journal of Labelled Compounds and Radiopharmaceuticals 2011, 54, 418-425. Still further processes for the preparation and/or purification of apixaban being provided in WO 2014/108919 A2, WO 2016/067308 A1, Research Disclosure database number 625018 (www.researchdisclosure .com), IP.com Database with IP.com number IPCOM000240694D, and WO 2014/203275 A2.

The known approaches for preparing apixaban involve numerous preparation steps. In these preparation procedures involving numerous preparation steps each single preparation step is of essential importance for the preparation of the desired final product, and therefore has to provide at least acceptable yields. Furthermore, in every single preparation step, the formation of side products has to be avoided for obtaining the desired final product, apixaban, in particular apixaban in crystalline form, in both high purity and high yields.

Thus, although approaches for preparing apixaban are known in the art, there still remains a need in the art for procedures for preparing apixaban, especially apixaban polymorphic form N-1, which provide apixaban, in particular apixaban in crystalline form, especially apixaban polymorphic form N-1, and/or one or more of its intermediates in advantageous yields and high purity. Furthermore, the preparation procedures should apply readily available excipients and/or excipients associated with no or merely low safety risks. Moreover, the procedures for preparing apixaban and its intermediates should allow a large scale production. Furthermore, these procedures should be time-efficient, and should avoid time-extensive preparation procedures, at least during one or several of the preparation steps. Finally, the procedures should avoid the use of materials which are cost-intensive materials, as well as should apply at least in some steps environmentally friendly materials and/or materials associated with low security risk.

### Summary of the invention

The aspects, advantageous features and preferred embodiments of the present invention summarized in the following items, respectively alone or in combination, further contribute to solving the problem of the present invention:
1. A process for preparing apixaban of formula (V), 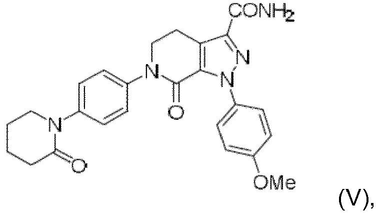 said process comprising:
   (i) providing a compound of formula (III), 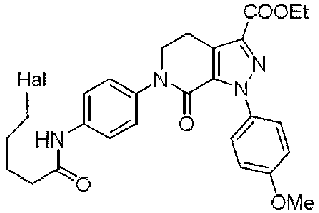 (III), wherein Hal is Br or Cl, preferably Cl;
   (ii) cyclizing the compound of formula (III) in the presence of potassium carbonate, a first solvent, and a phase transfer catalyst, to obtain a compound of formula (IV) 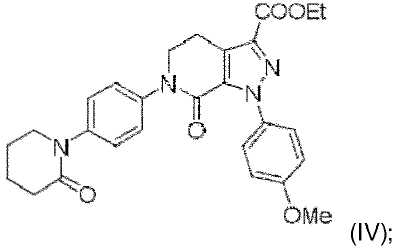
   (iii) amidating the compound of formula (IV) to obtain apixaban of formula (V); and
   (iv) optionally crystallizing apixaban of formula (V), especially optionally crystallizing apixaban polymorphic form N-1.
2. The process according to item 1, characterized in that the phase transfer catalyst is or comprises a phase transfer catalyst selected from the group of N,N,N,N-tetraalkylammonium halogenides, N-benzyl-N,N,N-trialkylammonium halogenides and mixtures thereof, preferably the phase transfer catalyst being or comprising a phase transfer catalyst selected from the group of N,N,N,N-tetraalkylammonium bromides and mixtures thereof, more preferably the phase transfer catalyst being or comprising N,N,N,N-tetrabutylammonium bromide.
3. The process according to any one of the preceding items, characterized in that the first solvent is or comprises acetonitrile.
4. The process according to any one of the preceding items, characterized in that step (iii) is or comprises reacting the compound of formula (IV) with NH₃ to obtain apixaban of formula (V), preferably at a temperature of 80°C or more,
   optionally characterized in that step (iii) is or comprises reacting a mixture comprising the compound of formula (IV) and methanol with NH₃ to obtain apixaban of formula (V), at a temperature of 80°C or more.
5. The process according to any one of the preceding items, wherein step (ii) comprises
   (ii-1) cyclizing the compound of formula (III) in the presence of a potassium carbonate, a first solvent, and a phase transfer catalyst, to prepare a reaction mixture comprising the compound of formula (IV),
   (ii-2) optionally subjecting the reaction mixture comprising the compound of formula (IV) to a removal of non-dissolved material from the reaction mixture, to obtain a solution comprising the compound of formula (IV),
   in particular optionally subjecting the reaction mixture comprising the compound of formula (IV) to filtration to obtain a solution comprising the compound of formula (IV),
   (ii-3) optionally removing the first solvent partially or completely from the solution comprising compound of formula (IV), and
   (ii-4) adding a second solvent and allowing precipitation of compound of formula (IV), optionally the second solvent is or comprises ethyl acetate,
   (ii-5) optionally isolating precipitate comprising or consisting of compound of formula (IV).
6. The process according to any one of the preceding items, wherein step (i) of providing a compound of formula (III) comprises:
   (A) contacting 3-chloro-1-(4-nitrophenyl)-5,6-dihydropyridin-2(1H)-one and ethyl 2-chloro-2-(2-(4-methoxyphenyl)hydrazono)acetate in the presence of a base to obtain a compound of formula (I) 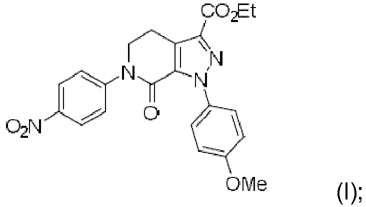
   (B) reducing the compound of formula (I) in the presence of hydrogen to obtain a compound of formula (II) 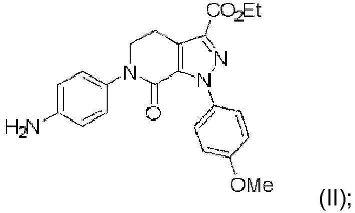
   (C) contacting the compound of formula (II) with 5-halogenovaleroyl halogenide to obtain a compound of formula (III), 5-halogenovaleroyl halogenide being 5-chlorovaleroyl chloride or 5-bromovaleroyl chloride, 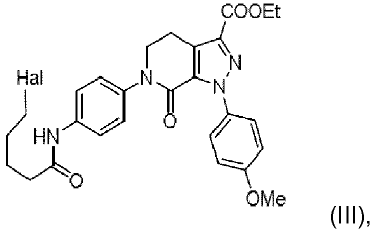 wherein Hal is Br or Cl.
7. The process according to item 6, characterized in that step (A) comprises (A-1) contacting 3-chloro-1-(4-nitrophenyl)-5,6-dihydropyridin-2(1H)-one and ethyl 2-chloro-2-(2-(4-methoxyphenyl)hydrazono)acetate in the presence of a base to obtain a reaction mixture comprising compound of formula (I),
   (A-2) allowing precipitation of the compound of formula (I) from the reaction mixture to obtain a mixture comprising a precipitate comprising or consisting of compound of formula (I),
   (A-3) isolating the precipitate comprising or consisting of compound of formula (I) from the mixture comprising a precipitate comprising or consisting of compound of formula (I),
   (A-4) contacting the isolated precipitate comprising or consisting of compound of formula (I) with water to obtain a mixture comprising water and compound of formula (I),
   (A-5) isolating compound of formula (I) from the mixture comprising water and compound of formula (I).
8. The process according to item 6, characterized in that step (B) is reducing the compound of formula (I) in the presence of hydrogen and a hydrogenation catalyst to obtain a compound of formula (II), optionally the hydrogenation catalyst being a hydrogenation catalyst comprising one or more metals selected from platinum group (in particular Pd or Pt) and Ni, especially a hydrogenation catalyst comprising Pd, further especially Pd/C, Pd/Al₂O₃, and mixtures thereof, or characterized in that step (B) is reducing the compound of formula (I) in the presence of hydrogen, a hydrogenation catalyst, and a solvent to obtain a compound of formula (II), optionally the solvent being or comprising N,N-dimethylformamide (DMF).
9. The process according to any one of the items 6 to 8, wherein step (B) comprises
   (B-1) reducing the compound of formula (I) in the presence of hydrogen to prepare a reaction mixture comprising compound of formula (II),
   (B-2) contacting the reaction mixture comprising compound of formula (II) with water to obtain a mixture comprising water and compound of formula (II),
   (B-3) optionally agitating, preferably stirring, the mixture comprising water and compound of formula (II),
   (B-4) isolating compound of formula (II) from the mixture comprising water and compound of formula (II).
10. The process according to any one of the preceding items, characterized in that step (iii) is or comprises reacting the compound of formula (IV) with NH₃ in the presence of a solvent to obtain apixaban of formula (V), optionally said solvent being or comprising a solvent selected from the group of alkyl alcohols, further optionally said solvent being or comprising methanol.
11. Method for the preparation of crystalline apixaban of formula (V), especially apixaban polymorphic form N-1, said method comprising
   (a) providing a mixture comprising apixaban of formula (V) and solvent at a first pressure and a first temperature, wherein the apixaban of formula (V) is at least partially present in not dissolved form at said first pressure and said first temperature in said mixture comprising apixaban of formula (V), and said solvent being methanol or a solvent mixture comprising or consisting of methanol and dichloromethane;
   (b) subjecting the mixture comprising apixaban of formula (V) and solvent to a second pressure and a second temperature, said second pressure being a pressure at least 100 pascal higher than the first pressure, optionally said second pressure being a pressure at least 1 kilopascal higher than the first pressure (further optionally said second pressure being a pressure at least 100 kilopascal (kPa) higher than the first pressure), preferably to obtain a clear solution;
   (c) optionally filtering the mixture obtained in step (b) to obtain a filtrate;
   (d) allowing crystallization of crystalline apixaban, especially apixaban polymorphic form N-1, in the mixture obtained in step (b) at a third temperature below said second temperature and/or at a third pressure below said second pressure, or allowing crystallization of crystalline apixaban of formula (V) in the filtrate obtained in optional step (c) at a third temperature below said second temperature and/or at a third pressure below said second pressure;
   (e) optionally isolating the crystalline apixaban of formula (V), especially apixaban polymorphic form N-1; and
   (f) optionally drying the isolated crystalline apixaban of formula (V), especially apixaban polymorphic form N-1.
12. Method for the preparation of crystalline apixaban of formula (V) according to item 11, characterized in that the crystalline apixaban is apixaban polymorphic form N-1.
13. Method for the preparation of crystalline apixaban of formula (V) according to any one of items 11 to 12, characterized in that said second pressure is a pressure in the range from 102 kilopascal to1300 kilopascal, optionally from 200 kilopascal to 1000 kilopascal.
14. Method for the preparation of crystalline apixaban of formula (V) according to any one of items 11 to 13, characterized in that the solvent is methanol, or a solvent mixture comprising or consisting of methanol and dichloromethane, optionally the solvent mixture having a weight ratio (methanol: dichloromethane) in the range of from 99 : 1 to 1 : 99, further optionally 70:30 to 90:10, preferably 80:20 to 90:10, or
   characterized in that the method comprises a step prior to step (a), said step prior to step (a) comprising:
   preparing apixaban of formula (V) in the presence of methanol.
15. Method for the preparation of crystalline apixaban of formula (V), especially apixaban polymorphic form N-1, said method comprising
   (a) providing a solution comprising apixaban of formula (V) dissolved in a solvent, said solvent being methanol or dichloromethane or a solvent mixture comprising or consisting of methanol and dichloromethane, optionally the solvent mixture having a weight ratio (methanol: dichloromethane) in the range of from 1:99 to 50:50, further optionally in the range of from 10:90 to 30:70;
   (b) adding methanol to said solution comprising apixaban of formula (V) (optionally said solution having a temperature in the range of from 15 to 40°C, further optionally in the range of from 25 to 35 °C), to precipitate crystalline apixaban of formula (V), wherein optionally methanol is added in such an amount to said solution comprising apixaban of formula (V) that the mixture obtained after the addition of methanol comprises apixaban of formula (V) and a solvent mixture comprising or consisting of methanol and dichloromethane, optionally the solvent mixture having a weight ratio (methanol:dichloromethane) in the range from 10:90 to 90:10, further optionally in the range of from 50:50 to 90:10, preferably 60:40 to 80:20;
   (c) isolating the precipitated crystalline apixaban of formula (V), especially apixaban polymorphic form N-1 (in particular from the mixture obtained after the addition of methanol); and
   (d) optionally slurrying the isolated crystalline apixaban of formula (V), especially apixaban polymorphic form N-1, in alcohol, optionally in alkyl alcohol, further optionally in C1-C5 alkyl alcohol, to obtain a slurry comprising the crystalline apixaban of formula (V);
   (e) optionally isolating the crystalline apixaban of formula (V), especially isolating apixaban polymorphic form N-1, from the slurry comprising the crystalline apixaban of formula (V), especially from the slurry comprising apixaban polymorphic form N-1;
   (f) optionally drying the isolated crystalline apixaban, especially drying the isolated apixaban polymorphic form N-1.

### Brief description of the Figures

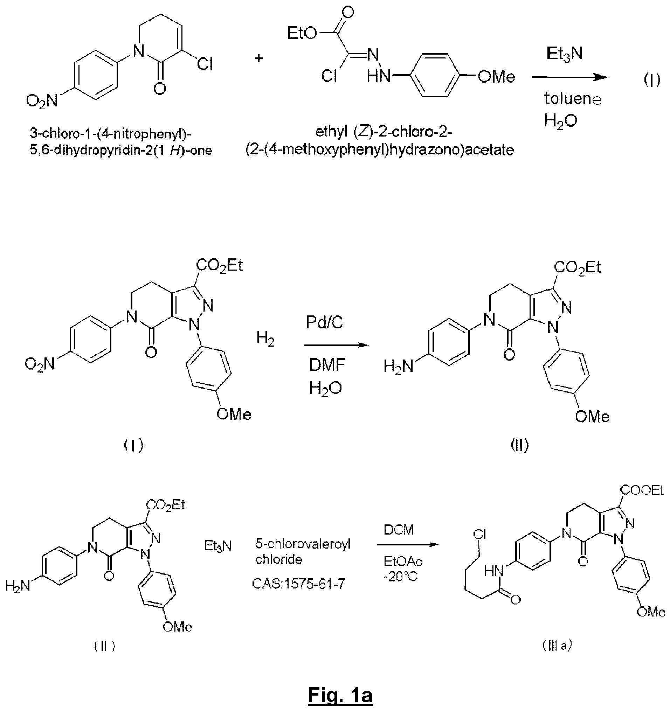

Figures 1A and 1B summarize several preparation steps of a preferred embodiment of the process of the present invention, and illustrate the apixaban preparation procedure described in the Example section.
Figure 2 illustrates the relationship between unreacted material and temperature, when reacting compound of formula (IV) with NH₃.

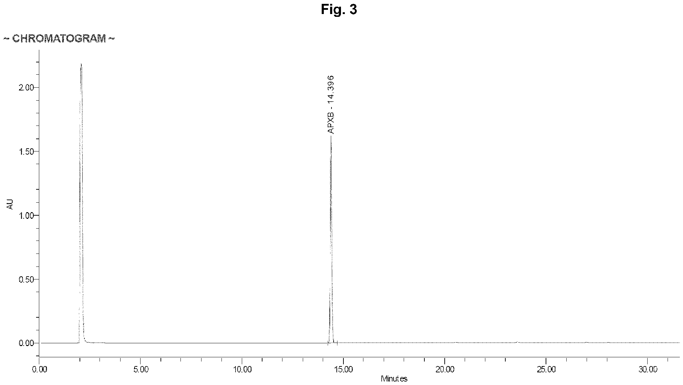

Figure 3 shows a chromatogram of the pilot batch synthesis described in Example 11.
Figure 4 illustrates the relationship between the concentration of compound of formula (IV) in the reaction mixture (amount of compound (IV) in mg per mL of reaction mixture), when reacting compound of formula (IV) with NH₃, and the formation of impurity AXN461 in the final product obtained (%AXN461).
Figure 5 illustrates the relationship between the concentration of ammonia (unit: M (mol/L) ammonia) and the purity of apixaban obtained (%APXB).
Figures 6-1 to 6-4 show material as obtained by the process described in example 10 characterized by PSD and SEM, before and after milling.

### Detailed description of the invention

The present invention relates to a process for preparing apixaban of formula (V), 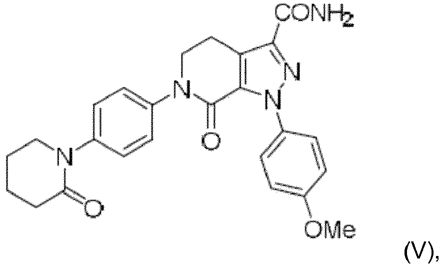 said process comprising:
(i) providing a compound of formula (III), 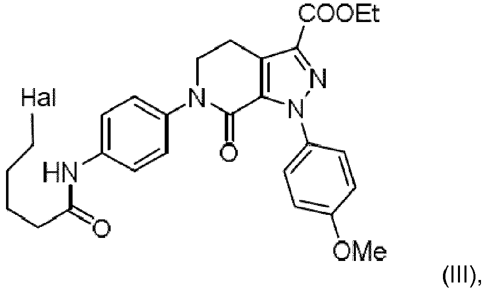 wherein Hal can be Br or Cl, preferably 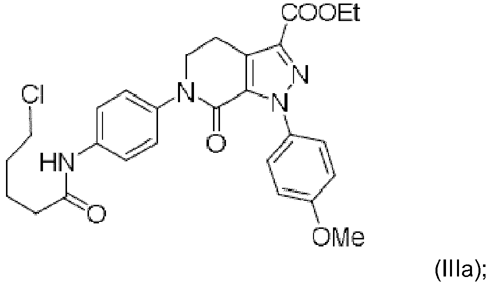
(ii) cyclizing the compound of formula (III) in the presence of potassium carbonate, a first solvent, and a phase transfer catalyst, to obtain a compound of formula (IV), 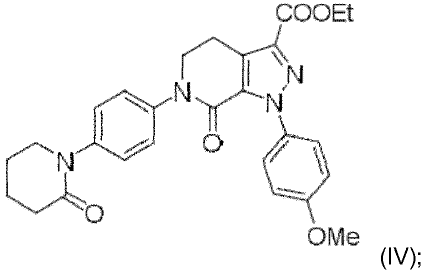
(iii) amidating the compound of formula (IV) to obtain apixaban of formula (V); and
(iv) optionally crystallizing apixaban of formula (V), especially optionally crystallizing apixaban polymorphic form N-1.

Step (ii) of the process for preparing apixaban can comprise:
(ii-1) cyclizing the compound of formula (III) in the presence of potassium carbonate, a first solvent, and a phase transfer catalyst (e.g. at a temperature in the cyclization reaction temperature range, as defined herein), to prepare a reaction mixture comprising the compound of formula (IV),
(ii-2) optionally subjecting the reaction mixture comprising the compound of formula (IV) to a removal of non-dissolved material (especially if present) from the reaction mixture, to obtain a solution comprising the compound of formula (IV), in particular optionally subjecting the reaction mixture comprising the compound of formula (IV) to filtration to obtain a solution comprising the compound of formula (IV),
(ii-3) optionally removing the first solvent partially or completely from the solution (in particular the solution obtained after step (ii-2)) comprising compound of formula (IV) or from the reaction mixture (obtained after step (ii-1)), and
(ii-4) adding a second solvent and allowing precipitation of compound of formula (IV), optionally the second solvent is or comprises ethyl acetate,
(ii-5) optionally isolating precipitate comprising or consisting of compound of formula (IV).

Surprisingly, the process of the present invention considerably improves the known approaches for preparing apixaban. Unexpectedly, cyclizing the compound of formula (III) in the presence of potassium carbonate, a first solvent, and a phase transfer catalyst, provides mild reaction conditions, is simple in operation, easy in purification, inexpensive in production costs, environmentally friendly, and suitable for industrial production. Without wishing to be bound to any theory, it is assumed that the presence of potassium carbonate surprisingly reduces the formation of degradation products, and thereby essentially contributes to the preparation of the cyclic intermediate (IV) in high yields and with high purity (e.g. with a purity of more than 99%). Furthermore, potassium carbonate controls the presence of water in the reaction mixture in a highly advantageous and mild manner. Moreover, potassium carbonate is an environmentally friendly compound and can contribute to the provision of a cost-effective provision of apixaban. In addition thereto, cyclizing the compound of formula (III) in the presence of potassium carbonate and a phase transfer catalyst to obtain a compound of formula (IV) provides a time-efficient reaction procedure and therefore contributes to a time-efficient production of apixaban. Finally, the combination of potassium carbonate and a phase transfer catalyst provides the advantage that this combination is effective for cyclizing the compound of formula (III), but at the same time is sufficiently mild for avoiding or minimizing side reactions. The combination of potassium carbonate and a phase transfer catalyst therefore provides the advantage that the formation of impurities can be avoided or minimized and the need for purification procedures, especially the need for complex, material-intensive, and time-intensive purification procedures, such as column chromatography, may be avoided.

In particular, the phase transfer catalyst can be or can comprise a phase transfer catalyst selected from the group of quaternary ammonium salts such as N,N,N,N-tetraalkylammonium halogenides or N-benzyl-N,N,N-trialkylammonium halogenides and mixtures thereof. Especially, the phase transfer catalyst can be or can comprise a phase transfer catalyst selected from the group of N,N,N,N-tetraalkylammonium halogenides or N-benzyl-N,N,N-trialkylammonium halogenides, and mixtures thereof, wherein alkyl can be selected from methyl, ethyl, propyl, butyl, pentyl, (and wherein optionally halogenide can be selected from bromide and chloride).

Preferably, the phase transfer catalyst can be or comprise a phase transfer catalyst selected from the group of N,N,N,N-tetraalkylammonium bromides, and mixtures thereof.

More preferably, the phase transfer catalyst can be or comprise a phase transfer catalyst selected from the group of N,N,N,N-tetraalkylammonium bromides, and mixtures thereof, wherein alkyl can be selected from methyl, ethyl, propyl, butyl, pentyl. More preferably, the phase transfer catalyst can be or comprise N,N,N,N-tetrabutylammonium bromide.

The step of cyclizing the compound of formula (III) in the presence of potassium carbonate, a first solvent, and a phase transfer catalyst can in particular comprise preparing a mixture comprising compound of formula (III), potassium carbonate, a first solvent, and a phase transfer catalyst. This mixture can be subjected to a temperature in the cyclization reaction temperature range, as defined herein.

In particular, step (ii) of cyclizing the compound of formula (III) in the presence of potassium carbonate, a first solvent, and a phase transfer catalyst can be or comprise preparing a mixture (e.g. in a reactor) comprising ethyl 6-(4-(5-chloropentanamido)phenyl)-1-(4-methoxyphenyl)-7-oxo-4,5,6,7-tetrahydro-1H-pyrazolo[3,4-c]pyridine-3-carboxylate (III), and acetonitrile. Potassium carbonate and N,N,N,N-tetrabutylammonium bromide can be added to this mixture.

The suspension can be stirred (e.g. under inert atmosphere, such as nitrogen atmosphere), e.g. at 75°C ± 5°C, (e.g. for 20 h ± 5 h). Subsequently, salts can be removed by filtration (e.g. hot filtration). Product can be precipitated from the filtrate by solvent exchange with ethyl acetate (e.g. at 65°C± 5°C). Solvent exchange with ethyl acetate can be in particular partially evaporating solvent from the filtrate, and adding ethyl acetate after partial evaporation of the solvent from the filtrate. Product can be isolated by filtration. Beige solid can be obtained (e.g. in a yield of 91% or more). The product can be used for the next step without further purification. This process step provides mild reaction conditions, and is furthermore simple in operation, easy in purification, inexpensive in production cost, environmentally friendly, and suitable for industrial production. Water in reaction mixture can be controlled by drying of potassium carbonate and stirring under inert atmosphere.

The first solvent can be an aprotic solvent, especially a polar aprotic solvent. The first solvent can be in particular selected from the group consisting of acetonitrile, acetone, N,N-dimethylformamide, tetrahydrofurane, ethyl acetate, dichloromethane, and mixtures thereof. In particular, the first solvent can be or comprise acetonitrile. The first solvent can for example comprise at least 50 wt.%, preferably at least 95 wt.% of acetonitrile, based on the total weight of the first solvent.

The term potassium carbonate can be a compound having the formula K₂CO₃. In particular, the term potassium carbonate encompasses all forms of potassium carbonate, including crystalline and amorphous forms thereof, as well as solutions thereof. The term potassium carbonate can comprise solvate(s), especially hydrate(s) thereof; in a preferred embodiment, potassium carbonate is potassium carbonate free of hydrate(s) of potassium carbonate.

In particular, milled, especially fine milled, potassium carbonate can be used, as a small particle size contributes to an advantageous solubility in solvent, especially acetonitrile. For example, potassium carbonate can be used having an average particle size below 30 µm, especially between 20 and 50 µm, e.g. between 20 and 40 µm. The average particle size can be determined by laser method using a Malvern Mastersizer.

The term "average particle size" as used herein refers to volume mean diameter of particles. The diameter and volume mean diameter can be determined by laser light scattering using e.g. a Malvern Mastersizer Aparatus. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer instrument.

Compound of formula (III) is a compound known in the art, and can be e.g. prepared as described in the experimental part of the present application.

Step (i) of the process for preparing apixaban can be providing a compound of formula (III) in pure form or as a material containing high amounts of compound of formula (III), such as e.g. a material containing 80 wt.% or more, or 90 wt.% or more, of compound of formula (III), but can be also providing a mixture, especially a reaction mixture, comprising compound of formula (III).

The second solvent can be in particular selected from the group consisting of esters, alcohols, aromatic hydrocarbons, and mixtures thereof. Esters can be in particular selected from compounds of formula R¹-C(O)-OR², wherein R¹ can be alkyl having 1 or 2 or 3 or 4 or 5 or 6 carbon atoms, and R² can be alkyl having 1 or 2 or 3 or 4 or 5 or 6 carbon atoms. Alcohols can be particular selected from compounds of formula R³-OH, R³ can be alkyl having 1 or 2 or 3 or 4 or 5 or 6 carbon atoms. Aromatic hydrocarbons can be selected from group of compounds that contains one or more aromatic benzene ring, most preferably one benzene ring. Especially, aromatic hydrocarbons can be selected from group of aromatic hydrocarbons having a mono- or bicyclic aromatic ring with 5 to 10 ring atoms, wherein optionally at one or two or three of the ring atoms -H is substituted with -CH₃, e.g. toluene.

Step (ii) of cyclizing the compound of formula (III) in the presence of potassium carbonate, a first solvent, and a phase transfer catalyst can be carried out at a temperature in the cyclization reaction temperature range. The cyclization reaction temperature range can be the temperature range above 28°C, e.g. the range of from 50°C to 90°C, especially the range of from 60°C to 82°C, further especially the range of from 71-77°C, further especially the range of from 74-76°C.

Step (ii) can be cyclizing the compound of formula (III) in the presence of potassium carbonate, a first solvent, and a phase transfer catalyst, to obtain a compound of formula (IV), compound of formula (III) and potassium carbonate being optionally present in a weight ratio between compound of formula (III) and potassium carbonate (compound of formula (III): potassium carbonate), which can be from 1.1:1 to 5:1, preferably 2:1 to 5:1, especially 2:1 to 4:1, most preferably about 2.53 : 1.

Step (ii) can be cyclizing the compound of formula (III) in the presence of potassium carbonate, a first solvent, and a phase transfer catalyst, to obtain a compound of formula (IV), phase transfer catalyst and potassium carbonate being optionally present in a weight ratio between phase transfer catalyst and potassium carbonate (phase transfer catalyst: potassium carbonate), which can be from 1:5 to 1:33, preferably 1:10 to 1:15, especially 1:11 to 1:14, most preferably about 1 : 12.9.

Step (iii) of the process for preparing apixaban can be or comprise reacting the compound of formula (IV) with NH₃ to obtain apixaban of formula (V), optionally at a temperature of 52°C or more (e.g. at a temperature of 52°C to 110°C), further optionally at a temperature of 80°C or more (e.g. at a temperature of 80°C to 110°C) and in the presence of an organic solvent (which solvent is also referred to hereinafter as organic solvent of step (iii)).

Preferably, the organic solvent of step (iii) can be an organic solvent selected from the group of protic solvents, especially selected from the group of alkyl alcohols, and mixtures thereof.

In particular, the alkyl alcohol can be selected from the group of alkyl alcohols having 1 or 2 or 3 or 4 or 5 carbon atoms. Especially, the organic solvent of step (iii) can be methanol.

Furthermore, step (iii) can be or comprise reacting the compound of formula (IV) with NH₃ in the presence of a solvent to obtain apixaban of formula (V), optionally said solvent being or comprising a protic solvent, especially being or comprising a solvent selected from the group of alkyl alcohols (e.g. containing 1 or 2 or 3 or 4 or 5 carbon atoms), further optionally said solvent being or comprising methanol.

When reacting the compound of formula (IV) with NH₃ in the presence of a solvent, said solvent can be free of water or can contain less than 0.1 wt.-% of water, preferably not more than 0.05 wt.-% of water, based on the weight of the solvent. Preferably, the reaction mixture comprising compound of formula (IV), NH₃, and a solvent can be free of water or can contain less than 0.1 wt.-% of water, preferably not more than 0.05 wt.-% of water, based on the weight of the reaction mixture.

NH₃ can be introduced in gaseous or liquid form into the reaction mixture. This can provide the advantage that the amount of ammonia introduced corresponds to the pressure of the reaction, and thereby influences the conversion of the reaction. Alternatively, NH₃ can be introduced in dissolved form, e.g. an organic solution (such as a solution comprising or consisting of NH₃ and one or more organic solvent(s)), into the reaction mixture. An organic solvent can be in particular a solvent which comprises at least one carbon atom and is liquid at 10°C (especially at 10°C and a pressure of 1 atm).

The concentration of starting material (intermediate of formula (IV)) in the reaction mixture, especially in reaction mixtures comprising NH₃, can surprisingly influence the performance of the reaction. Thus, the low solubility of the product at low temperatures provides high yields and an advantageous reaction time period also in more diluted reaction mixtures. Furthermore, carrying out the reaction of step (iii) with methanol and NH₃ can advantageously contribute to minimizing impurities, such as in particular:

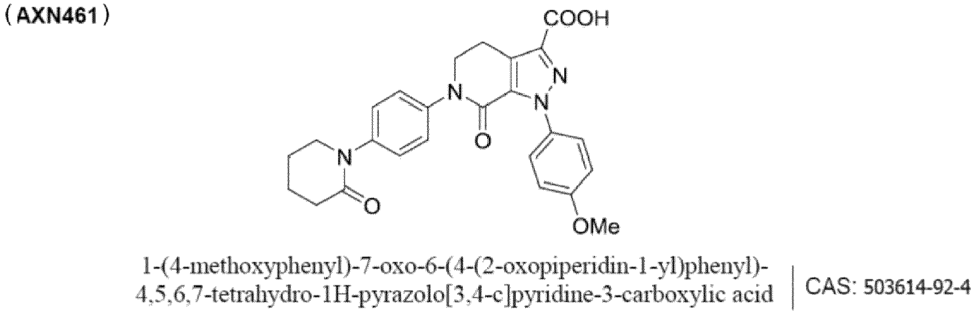

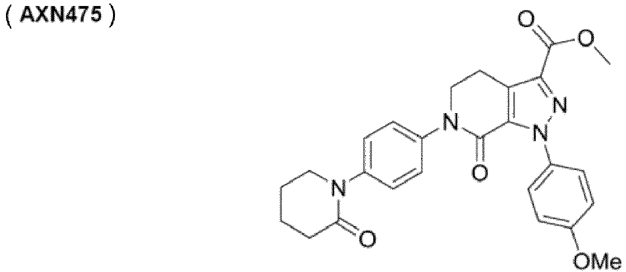

### methyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate

In particular, step (iii) of the process for preparing apixaban can be or comprise preparing a reaction mixture comprising an organic solvent (which solvent is also referred to herein as organic solvent of step (iii)) and compound of formula (IV), said organic solvent preferably comprising methanol), adding NH₃ (in gaseous form, liquid form and/or dissolved in a solvent capable of dissolving NH₃ (e.g. organic solvent(s), such as alkyl alcohols), preferably NH₃ in gaseous or liquid form), reacting the compound of formula (IV) with NH₃ to obtain apixaban of formula (V), optionally at a temperature of 52°C or more (e.g. at a temperature of 52°C to 110°C), further optionally at a temperature of 80°C or more (e.g. at a temperature of 80°C to 110°C) and in the presence of an organic solvent.

After step (iii), apixaban of high purity, especially apixaban in polymorphic form N-1 can be isolated (directly) from the reaction mixture. Optionally, the reaction mixture obtained after step (iii) can be cooled to a temperature below reaction temperature to induce the precipitation of apixaban, especially of apixaban in crystalline form, further especially of apixaban in polymorphic form N-1. This provides the advantage that apixaban of high purity can be obtained, as impurities remain dissolved in the reaction mixture, whereas apixaban, especially apixaban in crystalline form, further especially apixaban in polymorphic form N-1, precipitates. Preferably, the cooling (in particular of the reaction mixture obtained after step (iii) and/or the mixture for crystallizing apixaban during optional step (iv)) can comprise cooling the mixture (especially reaction mixture) at a cooling rate in the range of 0.1 to 1 K/minute, especially of 0.3 to 0.5 K/minute, further especially of 0.4 K /min. Especially, the cooling can comprise cooling the reaction mixture at a cooling rate in the range of 0.1 to 1 K/minute, especially of 0.3 to 0.5 K/minute, further especially of 0.4 K /min to a temperature in the range of 20 to 0°C, especially in the range of 10 to 0°C, optionally under agitation (e.g. stirring).

In particular, step (iii) can be or comprise preparing a mixture (e.g. in a reactor) comprising ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV), methanol and ammonia. Ammonia can be added in gas form and/or in liquid form (especially dissolved in alkyl alcohol, such as methanol). The so obtained mixture can be stirred (e.g. at 95°C± 5°C), e.g. for 10 h ± 2 h), especially at a pressure above ambient pressure (e.g. resulting from the addition of ammonia in gas form and/or in liquid form and reaction temperature higher than boiling point of mixture) and then cooled, e.g. to 5°C± 4°C. (Ambient pressure can be e.g. 1 atm.) The suspension can be optionally agitated (e.g. stirred (e.g. at that temperature (5°C± 4°C), e.g. for 1 h). Product can be isolated by filtration. Apixaban can be obtained as a product with high purity, e.g. all impurities can be HPLC area <0.10%, and in crystalline form (N-1 polymorphic form).

Preferably, step (iii) of amidating the compound of formula (IV) to obtain apixaban of formula (V) can be therefore: (iii) amidating the compound of formula (IV) to obtain apixaban of formula (V), said compound of formula (IV) being present in a mixture comprising methanol. In particular, step (iii) of the process for preparing apixaban can be or comprise preparing a reaction mixture comprising methanol and compound of formula (IV), adding NH₃ (preferably in gaseous form and/or in liquid form; especially dissolved in alkyl alcohol, such as methanol), and reacting the compound of formula (IV) with NH₃ to obtain apixaban of formula (V). Reacting the compound of formula (IV) with NH₃ can be preferably at a pressure above 1 atm (101 325 Pa), further optionally at a pressure in the range of 300 kPa to 1100 kPa. This allows taking advantage of the advantageous properties of methanol for dissolving apixaban, as will be discussed below.

Preferably, the temperature of the reaction mixture obtained after step (iii) of amidating the compound of formula (IV) to obtain apixaban of formula (V) (e.g. said temperature being a temperature of 80°C or more or a temperature of 90°C or more; preferably said reaction mixture comprising methanol) is decreased, preferably at a cooling rate in the range of 0.3 to 0.5 K/min e.g. of about 0.4 K/min, (e.g. to a temperature 10°C or less, such as 3 to 6°C) for precipitating apixaban in crystalline form, especially apixaban form N-1.

Furthermore, during the amidation step of the preparation method of the present invention, methanol can be used as solvent. When doing so, a surprisingly good potential of purification is observed, e.g. form N-1 with purity of 99.85% can be obtained.

The inventors surprisingly discovered that solubility of apixaban in methanol can be improved at high pressure and elevated temperature without formation of additional impurities. This is highly surprising, as methanol is usually considered by the skilled person as antisolvent for apixaban. For example, 1g of apixaban can be dissolved in 15 mL of methanol under a pressure of 200 kPa and at a temperature of 90°C while its solubility at atmospheric pressure and reflux temperature is only 8.67 mg in 1 mL. Apixaban crystallizes upon cooling of the solution, and N-1 form, especially N-1 form of high purity is obtained. Furthermore, N-1 form prepared as described herein can be dried without problems, and achieves usual requirements for residual solvents.

Preferably, step (iii) of amidating the compound of formula (IV) to obtain apixaban of formula (V) can be therefore step (iii) of amidating the compound of formula (IV) to obtain apixaban of formula (V), said compound of formula (IV) being present in a mixture comprising methanol. In particular, step (iii) of process for preparing apixaban can be or comprise preparing a reaction mixture comprising methanol, NH₃ and compound of formula (IV), (e.g. by preparing a reaction mixture comprising methanol and compound of formula (IV), adding NH₃ (preferably in gaseous form and/or dissolved in methanol)), and reacting the compound of formula (IV) with NH₃ to obtain apixaban of formula (V). Preferably, the temperature of the reaction mixture comprising methanol obtained after step (iii) of amidating the compound of formula (IV) to obtain apixaban of formula (V) (e.g. said temperature being a temperature of 80°C or more or a temperature of 90°C or more) is decreased, preferably at a cooling rate in the range of 0.3 to 0.5 K/min e.g. of about 0.4 K/min, (e.g. to a temperature 10°C or less, such as 3 to 6°C) for precipitating apixaban in crystalline form, especially apixaban form N-1.

In particular, step (iii) can be or can comprise reacting the compound of formula (IV) with NH₃ in the presence of alkyl alcohol to obtain apixaban of formula (V), wherein said reacting of the compound of formula (IV) with NH₃ in the presence of alkyl alcohol to obtain apixaban of formula (V) is or comprises:
(iii-a.) preparing a mixture comprising compound of formula (IV), NH₃, and alkyl alcohol, said mixture having a ratio between NH₃ and alkyl alcohol (NH₃: alkyl alcohol) which is in the range of at least 4 mol NH₃: 1 L alkyl alcohol, optionally is in the range 4 mol NH₃: 1 L alkyl alcohol to 12 mol NH₃: 1 L alkyl alcohol, (optionally the ratio between NH₃ and alkyl alcohol (NH₃: alkyl alcohol) can be in the range 6 mol NH₃: 1 L alkyl alcohol to 12 mol NH₃: 1 L alkyl alcohol, further optionally in the range 8 mol NH₃: 1 L alkyl alcohol to 12 mol NH₃: 1 L alkyl alcohol),
   optionally said mixture comprising less than 0.1 wt.-% of water, further optionally said mixture comprising not more than 0.05 wt.-% of water;
(iii-b.) subjecting the mixture obtained in step (iii-a.) to a temperature of 52°C or more (e.g. to a temperature in the range from 52°C to 110°C), optionally to a temperature of 80°C or more (e.g. to a temperature in the range from 80°C to 110°C), and allowing reacting of compound of formula (IV) with NH₃ to obtain apixaban of formula (V), (optionally step (iii-b.) being carried out at a pressure above 1 atm (101 325 Pa), e.g. at a pressure from 102 kilopascal to 1300 kilopascal, optionally from 200 kilopascal to 1000 kilopascal);
(iii-c.) decreasing the temperature of the mixture obtained in step (iii-b.), optionally to a temperature of 25°C or less, optionally to a temperature in the range from 24°C to 2°C, further optionally to a temperature in the range from 22°C to 4°C, further optionally to a temperature in the range from 10°C to 5°C (said decreasing of the temperature can be for example during 2-24 h, especially during 2-10 h), and allowing precipitation of apixaban of formula (V), in particular apixaban comprising or consisting of apixaban form N-1; and
(iii-d.) optionally isolating apixaban, in particular apixaban comprising or consisting of apixaban form N-1;
optionally, the alkyl alcohol can be selected from the group of methanol, ethanol, propanol (especially straight chain or branched propanol), butanol (especially straight chain or branched butanol), pentanol (especially straight chain or branched pentanol). Preferably, the alkyl alcohol is methanol.

Optionally, step (iii-c.) is or comprises decreasing the temperature of the mixture obtained in step (iii-b.), the temperature of the mixture obtained in step (iii-b.) decreasing less than 2°C per minute. Further optionally, step (iii-c.) can be or comprise decreasing the temperature of the mixture obtained in step (iii-b.) at a rate in the range 0.02 to 2 K/min, in particular at a rate in the range of 0.1 to 1 K/min, especially at a rate in the range of 0.15 to 0.5 K/min. Optionally, the mixture obtained in step (iii-a.) can have a content of 40 to 150 mg of compound of formula (IV) per milliliter of the reaction mixture, especially a content of 43 to 100 mg of compound of formula (IV) per milliliter of the reaction mixture, especially a content of 45 to 55 mg of compound of formula (IV) per milliliter of the reaction mixture, in particular a content of 49 to 51 mg (e.g. about 50 mg) of compound of formula (IV) per milliliter of the reaction mixture (The volume of the reaction mixture can be determined at 20°C, especially at a temperature of 20°C and a pressure of 1 atm (101 325 Pa)).

Optionally, during step (iii-c.) the pressure optionally applied during step (iii-b.) is adapted to ambient pressure. Ambient pressure can be the pressure present at the place where the method is carried out. Ambient pressure can be for example a pressure in the range of 80 to 102 kPa, especially in the range of 95 to 101kPa, further especially in the range of 97 to 101 kPa.

In one embodiment, polymorph N-1 is obtained by filtration of reaction mixture (e.g. the reaction mixture obtained in step (iii) of amidating the compound of formula (IV) to obtain apixaban of formula (V)), at a temperature higher than 70°C, and at a pressure between 6-10 bar (600-1000 kPa), and precipitation of product by cooling. This embodiment provides advantageously high yields and polymorph N-1 in high purity.

In particular, the method can comprise a step prior to step (a) comprising: preparing apixaban of formula (V) in the presence of methanol.

Step (i) of providing a compound of formula (III) can comprise:
(A) contacting 3-chloro-1-(4-nitrophenyl)-5,6-dihydropyridin-2(1H)-one and ethyl 2-chloro-2-(2-(4-methoxyphenyl)hydrazono)acetate (especially ethyl (Z)-2-chloro-2-(2-(4-methoxyphenyl)hydrazono)acetate) in the presence of a base to obtain a compound of formula (I) 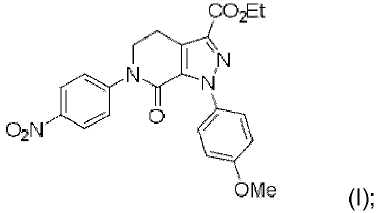
(B) reducing the compound of formula (I) in the presence of hydrogen to obtain a compound of formula (II) 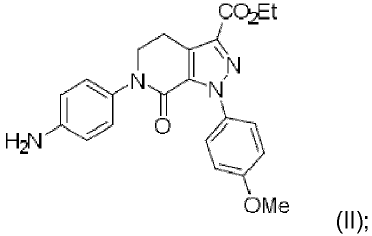
(C) contacting the compound of formula (II) with 5-halogenovaleroyl halogenide to obtain a compound of formula (III), 5-halogenovaleroyl halogenide being 5-chlorovaleroyl chloride or 5-bromovaleroyl chloride, 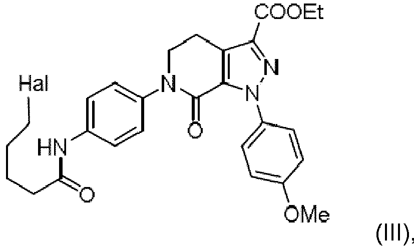 wherein Hal is Br or Cl,
   optionally contacting the compound of formula (II) with 5-chlorovaleroyl chloride to obtain a compound of formula (IIIa)

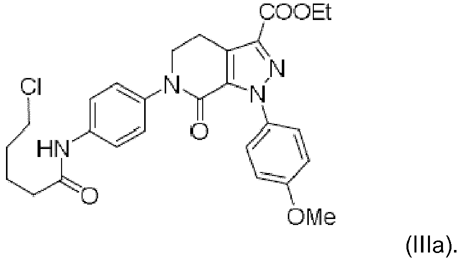

In an embodiment, step (C) can be or comprise contacting the compound of formula (II) with 5-bromovaleroyl chloride to obtain a compound of formula (III), 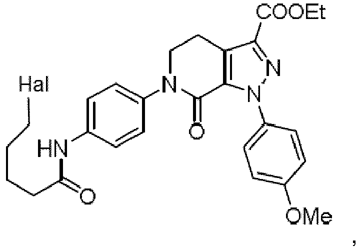 wherein Hal is Br.

In particular, step (A) of contacting 3-chloro-1-(4-nitrophenyl)-5,6-dihydropyridin-2(1H)-one and ethyl 2-chloro-2-(2-(4-methoxyphenyl)hydrazono)acetate in the presence of a base to obtain a compound of formula (I) can comprise
(A-1) contacting 3-chloro-1-(4-nitrophenyl)-5,6-dihydropyridin-2(1H)-one and ethyl 2-chloro-2-(2-(4-methoxyphenyl)hydrazono)acetate in the presence of a base (optionally at a temperature in the range 60 to 110 °C) to obtain a reaction mixture comprising compound of formula (I), wherein said base optionally can be or comprise a trialkylamine, such as triethylamine,
(A-2) allowing precipitation of the compound of formula (I) from the reaction mixture to obtain a mixture comprising a precipitate comprising or consisting of compound of formula (I),
(A-3) isolating the precipitate comprising or consisting of compound of formula (I) from the mixture comprising a precipitate comprising or consisting of compound of formula (I),
(A-4) contacting the isolated precipitate comprising or consisting of compound of formula (I) with water to obtain a mixture comprising water and compound of formula (I),
(A-5) isolating compound of formula (I) from the mixture comprising water and compound of formula (I).

Ethyl 2-chloro-2-(2-(4-methoxyphenyl)hydrazono)acetate can be especially ethyl (Z)-2-chloro-2-(2-(4-methoxyphenyl)hydrazono)acetate).

Step (A) can in particular comprise preparing a mixture (e.g. in a reactor) comprising toluene, 3-chloro-1-(4-nitrophenyl)-5,6-dihydropyridin-2(1H)-one, ethyl (Z)2-chloro-2-(2-(4-methoxyphenyl)hydrazono)acetate. Triethylamine can then be added (e.g. dropwise) for example e.g. a temperature above 20 °C, such as 75°C. After the addition, reaction mixture can be stirred (e.g. at 75°C ± 10°C), for example for 5 h. After the reaction is completed, reaction mixture can be cooled (e.g. to 5°C± 4°C). The product can be isolated, e.g. by filtration, and slurried in water (e.g. at 20°C± 5°C). Yellow solid can be obtained (e.g. at a yield of about 90% or more). The product can be used for the next step without further purification. This procedure provides an uncomplicated approach to the desired intermediate. The product can be obtained with filtration. Salt can be removed by slurrying in water. This process shows a good potential of purification, for example product can be obtained with purity more than 99.9%. Crude product can be isolated, especially in one phase system. Both isolation and slurrying in water can be made in the same container, e.g. in a filter dryer. Due to the high purity achieved, there is no need for recrystallization or slurrying in organic solvent. Optionally, recrystallization or slurrying in organic solvent can be carried out, if desired.

3-chloro-1-(4-nitrophenyl)-5,6-dihydropyridin-2(1H)-one and ethyl (Z)-2-chloro-2-(2-(4-methoxyphenyl)hydrazono)acetate are compounds known in the art and are described e.g. in WO 2007/001385 A2.

Step (B) can be in particular reducing the compound of formula (I) in the presence of hydrogen and a hydrogenation catalyst to obtain a compound of formula (II). In particular, the hydrogenation catalyst can be or comprise a hydrogenation catalyst comprising at least one metal selected from the platinum group (e.g. one or more of Pd, Pt) or Ni, especially the hydrogenation catalyst can be or comprise a hydrogenation catalyst selected from the group of hydrogenation catalysts comprising palladium, and mixtures thereof, further especially the hydrogenation catalyst can be or comprise especially Pd/C, Pd/Al₂O₃, and mixtures thereof.

Especially, step (B) can be reducing the compound of formula (I) in the presence of hydrogen, a hydrogenation catalyst, and a solvent to obtain a compound of formula (II). Furthermore, step (B) can be reducing the compound of formula (I) in the presence of hydrogen, a hydrogenation catalyst, and a solvent to obtain a compound of formula (II), the solvent being or comprising N,N-dimethylformamide (DMF).

In particular, step (B) can comprise:
(B-1) reducing the compound of formula (I) in the presence of hydrogen to prepare a reaction mixture comprising compound of formula (II), optionally at a temperature in the range of from 20 to 60°C, e.g. in the range from 40 to 50°C,
(B-2) contacting the reaction mixture comprising compound of formula (II) with water to obtain a mixture comprising water and compound of formula (II),
(B-3) optionally agitating, preferably stirring, the mixture comprising water and compound of formula (II),
(B-4) isolating compound of formula (II) from the mixture comprising water and compound of formula (II).

Step (B) of reducing the compound of formula (I) in the presence of hydrogen to obtain a compound of formula (II), especially step (B-1), can in particular be or comprise preparing a mixture comprising ethyl 1-(4-methoxyphenyl)-6-(4-nitrophenyl)-7-oxo-4,5,6,7-tetrahydro-1H-pyrazolo[3,4-c]pyridine-3-carboxylate (I), and N,N-Dimethylformamide. Under an inert atmosphere (e.g. the reactor containing the mixture can be inertised), and a catalyst comprising palladium (e.g. 5% Pd/C) is added. Reaction mixture can be stirred under hydrogen (e.g. at 45°C± 5°C), for example for 5 h. After the reaction is completed, catalyst can be removed by filtration. The product can be precipitated from the so obtained filtrate by (e.g. dropwise) addition to water (e.g. at 50°C± 5°C). Suspension can be stirred (e.g. at 50°C ± 5°C) (e.g. for 1 h) and then cooled (e.g. to 5°C± 4°C). Product can be isolated by filtration (e.g. in a filter dryer), and DMF can be (optionally) removed by slurring in water. Yellow crystalline solid can be obtained. The product can be used for the next step without further purification and drying. Precipitating a product by a dropwise addition of the reaction mixture into warm water at (e.g. at 50°C± 5°C), such as at about 50°C, allows to obtain a product with advantageous crystallinity. Product can be isolated (e.g. on a filter dryer); optionally, the product obtained can be slurried in water to remove residual DMF.

Step (C) of contacting the compound of formula (II) with 5-halogenovaleroyl chloride to obtain a compound of formula (III), can in particular be or comprise preparing a mixture comprising ethyl 6-(4-aminophenyl)-1-(4-methoxyphenyl)-7-oxo-4,5,6,7-tetrahydro-1H-pyrazolo[3,4-c]pyridine-3-carboxylate (II), and methylene chloride. The reaction mixture can be stirred (e.g. at RT, such as e.g. at 20°C± 5°C, until solid is dissolved. Reaction mixture can then be cooled (e.g. to 15°C ± 5°C), and N,N,N-triethylamine can be added, e.g. dropwise. Subsequently, 5-chlorovaleroyl chloride can be added, e.g. dropwise. Reaction mixture can be stirred (e.g. for 1 h ± 30 minutes). Subsequently, temperature can be raised (e.g. to 22°C ± 5°C), and the water can be added. Phases can be separated, and the product can be precipitated from the organic phase by addition of ethyl acetate. The so obtained suspension can be stirred (e.g. at 55°C ± 10°C) (e.g. for 1 h ± 30 minutes) and cooled (e.g. to -20°C ± 10°C). Product can be isolated by filtration. Beige solid can be obtained in advantageous yields (e.g. a yield 93% or more). The product can be used for the next step without further purification.

During this process step, a solvent exchange can be avoided, as the product can be directly precipitated from the organic phase by addition of alkyl ester (e.g. ethyl acetate), especially alkyl ester (e.g. ethyl acetate) with high purity. This process is time-effective, and contributes to low production costs, as the product can be precipitated from the organic phase.

The present inventors surprisingly discovered that a mixture of DCM (dichloromethane) and MeOH (methanol) considerably increases the solubility of apixaban even at moderate or room temperatures. By addition of methanol, material can be precipitated, and pure form N-1 is obtained. Product can be further slurried in ethanol or other alcohol(s) to stabilize the polymorphic form, as well as to improve the removal of the residual solvents. In particular, the solvent-solute-ratio can be optionally advantageously decreased by subjecting apixaban containing mixtures to increased pressures and increased temperatures.

According to this another aspect, there is provided a method for the preparation of crystalline apixaban of formula (V), especially apixaban polymorphic form N-1, said method comprising:
(a) providing a mixture comprising apixaban of formula (V) and solvent at a first pressure and a first temperature,
   wherein the apixaban of formula (V) is at least partially present in non-dissolved form at said first pressure and said first temperature in said mixture comprising apixaban of formula (V), and
   said solvent being methanol or a solvent mixture comprising or consisting of methanol and dichloromethane;
(b) subjecting the mixture comprising apixaban of formula (V) and solvent to a second pressure and a second temperature, said second pressure being a pressure at least 100 pascal higher than the first pressure, optionally said second pressure being a pressure at least 1 kilopascal higher than the first pressure (further optionally said second pressure being a pressure at least 100 kilopascal (kPa) higher than the first pressure), preferably to obtain a solution;
(c) optionally filtering the mixture obtained in step (b) to obtain a filtrate;
(d) allowing crystallization of crystalline apixaban, especially apixaban polymorphic form N-1, in the mixture obtained in step (b) at a third temperature below said second temperature and/or at a third pressure below said second pressure, or
   allowing crystallization of crystalline apixaban of formula (V) in the filtrate obtained in optional step (c) at a third temperature below said second temperature and/or at a third pressure below said second pressure;
(e) optionally isolating the crystalline apixaban of formula (V), especially apixaban polymorphic form N-1; and
(f) optionally drying the isolated crystalline apixaban of formula (V), especially apixaban polymorphic form N-1.

Preferably, the method for the preparation of crystalline apixaban of formula (V), especially apixaban polymorphic form N-1, comprises decreasing the second temperature to the third temperature and/or decreasing the temperature of the optionally obtained filtrate to the third temperature at a cooling rate in the range of 0.1 to 1 K/minute, especially of 0.3 to 0.5 K/minute, further especially of 0.4 K /min; optionally during said decreasing the mixture or filtrate can be agitated, e.g. stirred.

In particular, the first pressure can be the pressure present at the place where the method is carried out e.g. a pressure of about 1 atm (101 325 Pa), e.g. a pressure in the range of 100 to 105 kPa (kilopascal). In particular, the first pressure can be a pressure in the range of 80 to 101 kPa, especially in the range of 95 to 101 kPa, further especially in the range of 100 to 101 kPa.

Furthermore, the first temperature can be a temperature in the range from10 °C to 35°C, optionally in the range from 25 °C to 30 °C.

In particular, the second pressure can be a pressure in the range from 102 kilopascal to 1300 kilopascal, optionally from 200 kilopascal to 1000 kilopascal.

Especially, the second temperature can be a temperature in the range from 52 °C to 110°C, optionally in the range from 80 °C to 110°C.

Especially, the third pressure can be a pressure in the same range as for the first pressure.

In particular, the third temperature can be a temperature in the range from 2 °C to 24°C, optionally in the range from 5°C to 10°C.

Especially, the solvent used in step (a) can be methanol or a solvent mixture comprising or consisting of methanol and dichloromethane.

Furthermore, the solvent used in step (a) can be methanol, dichloromethane, or a solvent mixture comprising or consisting of methanol and dichloromethane, optionally the solvent mixture having a weight ratio (methanol: dichloromethane) in the range of from 99 : 1 to 1 : 99, further optionally 70:30 to 90:10, preferably 80:20 to 90:10.

The term "apixaban" as used herein can relate to apixaban in crystalline form, and in amorphous form. Preferably, apixaban prepared by the methods and processes disclosed herein can be or comprise apixaban Form N-1.

Form N-1 (neat) and Form H2-2 (hydrate) of apixaban are known in the art and may be characterized by unit cell parameters substantially equal to the following shown in the following Table 1, published in WO 2011/106478 A2:

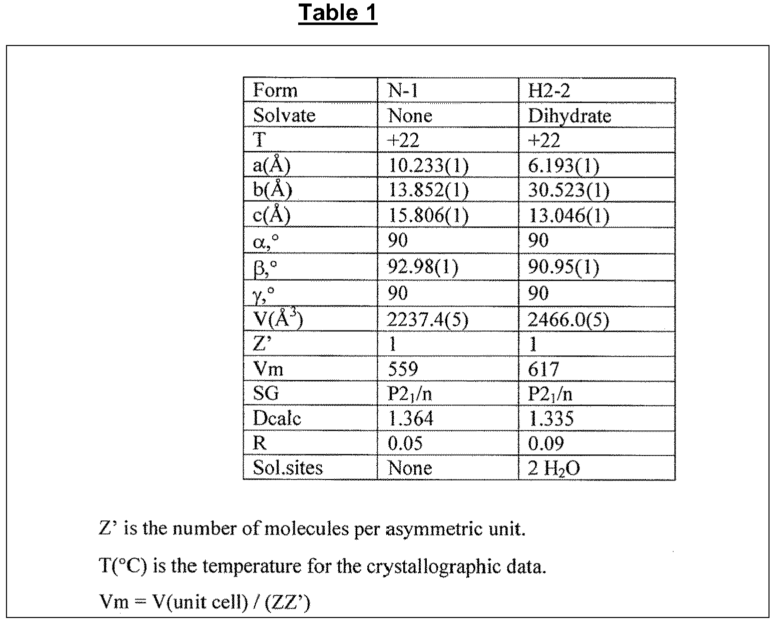

Furthermore, characteristic X-ray diffraction peak positions (degrees 2θ±0.1) at room temperature, based on a pattern (especially a high quality pattern) collected with a diffractometer (CuKα) (with a spinning capillary with 2θ calibrated with a NIST suitable standard), are provided in WO 2011/106478 A2 and in EP 2 538 925 B1 and are shown in Table 2 below for Form N-1 and H2-2, respectively. Thus, Form N-1 can be characterized by a X-ray diffraction pattern collected with a diffractometer (CuKα) showing the characteristic X-ray diffraction peak positions (degrees 2θ±0.1) shown in Table 2 below, and Form H2-2 can be characterized by a X-ray diffraction pattern collected with a diffractometer (CuKα) showing the characteristic X-ray diffraction peak positions (degrees 2θ±0.1) shown in Table 2 below:

**Table 2**

| Form N-1 | Form H2-2 |
|---|---|
| 10.0 | 5.8 |
| 10.6 | 7.4 |
| 12.3 | 16.0 |
| 12.9 | 20.2 |
| 18.5 | 23.5 |
| 27.1 | 25.2 |

Preferably, step (iv) of optionally crystallizing apixaban of formula (V), especially optionally crystallizing apixaban polymorphic form N-1, can consist of or can comprise any of the methods for the crystallization and/or purification of apixaban described herein, and in particular can consist of or can comprise steps (a) to (f) of the method(s) for the preparation of crystalline apixaban, especially apixaban Form N-1, described herein.

The crystalline apixaban of formula (V) obtained after one of or all of steps (d), (e), and (f) can be or can comprise apixaban polymorphic form N-1.

In particular, the method can comprise a step prior to step (a) comprising: preparing apixaban of formula (V) in the presence of methanol, optionally in the presence of methanol, and ammonia.

According to another aspect, there is provided a method for the preparation of crystalline apixaban of formula (V), especially apixaban polymorphic form N-1, said method comprising
(a) providing a solution comprising apixaban of formula (V) dissolved in a solvent, said solvent being methanol or dichloromethane or a solvent mixture comprising or consisting of methanol and dichloromethane, optionally the solvent mixture having a weight ratio (methanol: dichloromethane) in the range of from 1 : 99 to 50: 50, further optionally in the range of from 10 : 90 to 30: 70;
(b) adding methanol to said solution comprising apixaban of formula (V), said solution having a temperature in the range of from 15 to 40 °C, preferably in the range of from 25 to 35 °C, to precipitate crystalline apixaban of formula (V), wherein optionally methanol is added in such an amount to said solution comprising apixaban of formula (V) that the mixture obtained after the addition of methanol comprises apixaban of formula (V) and a solvent mixture comprising or consisting of methanol and dichloromethane, the solvent mixture optionally having a weight ratio (methanol:dichloromethane) in the range from 10:90 to 90:10, further optionally in the range of from 50:50 to 90:10, preferably 60:40 to 80:20;
(c) isolating the precipitated crystalline apixaban of formula (V), especially apixaban polymorphic form N-1; and
(d) optionally slurrying the isolated crystalline apixaban of formula (V), especially apixaban polymorphic form N-1, in alcohol, optionally in alkyl alcohol, further optionally in C1-C5 alkyl alcohol, to obtain a slurry comprising the crystalline apixaban of formula (V);
(e) optionally isolating the crystalline apixaban of formula (V), especially isolating apixaban polymorphic form N-1, from the slurry comprising the crystalline apixaban of formula (V), especially from the slurry comprising apixaban polymorphic form N-1;
(f) optionally drying the isolated crystalline apixaban, especially drying the isolated apixaban polymorphic form N-1.

In particular, the method for the preparation of crystalline apixaban of formula (V), especially apixaban polymorphic form N-1, can comprise preparing a mixture, usually solution, comprising apixaban, methylene chloride and methanol. The solution can be agitated (e.g.stirred), for example (e.g. at 35°C ± 5°C) (e.g. for 30 ± 10 min) and filtered. Apixaban can be precipitated with an addition of methanol (e.g. at room temperature (e.g. at 20°C ± 5°C). Suspension can be cooled in (e.g. 2h ± 30 min) (for example to -20°C ± 5°C) and stirred for 1 h ± 10 min). Product can be isolated (e.g. on a filter dryer). N-1 polymorphic form, especially in pure form, can be obtained. For stabilizing the polymorphic form and for improving the removal of residual solvents, the isolated product (e.g. in the form of a wet cake) can be slurried in ethanol (e.g. at 55°C ± 5°C) (for example for 5 hours ± 30 min). Suspension can then be cooled to room temperature and filtered. The so obtained wet cake can be dried (e.g. at a temperature in the range 50-80°C), preferably at a pressure below ambient pressure (e.g. at a pressure p=5± 0,5 kPa). Pure N-1 form can be obtained, having a purity of more than 99.90 %, as determined by HPLC.

An advantage of the method for the preparation of crystalline apixaban, especially apixaban polymorphic form N-1, is that the formation of impurities (e.g. dimer impurities, acid impurity and/or H-2 form) can be avoided, as well as that also presence of residual solvents such as 1,2-propandiol can be avoided. Furthermore, the method(s) and step(s) for the preparation of crystalline apixaban described herein may be carried out at comparatively low temperatures, as well as avoid the use of large quantities of 1,2-propandiol and/or water.

All described processes ensure filtration (especially GMP filtration) of apixaban solution, especially from small volumes of solvent(s), which solvent(s) can be mixture of DCM and methanol in the range from 1:99 to 99:1 or pure methanol.

In particular, the present inventors developed processes and methods providing apixaban purity >99.5%, presence of impurities of not more than 0.10%; pure polymorphic form N-1, with average particle size in range 20 µm to 400 µm, preferably 30µm to 200 µm, most preferably 40 µm to 100µm is prepared. The process of the present invention furthermore provides the advantage that apixaban can be directly isolated from reaction mixture after amidation; especially with controlled cooling conditions purity >99.9%, pure polymorphic form N-1 is obtained. The present inventors surprisingly discovered that particles obtained by this process are very fine elongated rods, and can be easy milled to an average particle size in the range from 2 to 50 µm, preferably in the range from 2 to 20 µm, most preferably in the range from 2 to 10 µm, for example with any type of milling equipment for dry or wet milling, such as jet mill, pin mill, high shear homogeniser. The dried, isolated crystalline apixaban, especially the dried isolated apixaban polymorphic form N-1, obtained after step (f) of any of the methods for the preparation of crystalline apixaban, can have an average particle size in the range of from 20 µm to 400 µm, preferably from 30µm to 200 µm, most preferably from 40 µm to 100µm.

The term "average particle size" as used herein refers to volume mean diameter of particles. The diameter and volume mean diameter can be determined by laser light scattering using e.g. a Malvern Mastersizer Aparatus. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer instrument.

Furthermore, pure polymorphic form N-1 is very well controlled by the process and overcomes problems known from other processes, like residual solvents, and/or H2-2 polymorphic form contamination. From that point of view claimed process is robust on industrial scale.

In particular, the method can comprise a step prior to step (a) comprising:
preparing apixaban of formula (V) in the presence of methanol.

### Methods

The purity of apixaban in general may be determined with the following HPLC method:

| | |
|---|---|
| **Column:** | YMC Triart C8 Plus, 150 x 4.6 mm, 3 mm particles |
| **Mobile Phase: Eluent A:** | 0.005 M NaH₂PO₄, pH=6.0 |
| **Eluent B:** | 90% acetonitrile |

**Gradient:**

| Time (min) | % v/v A | % v/v B |
|---|---|---|
| 0 | 80 | 20 |
| 3 | 80 | 20 |
| 22 | 19 | 81 |
| 27 | 19 | 81 |
| 30 | 80 | 20 |

| | |
|---|---|
| **Post time:** | 5 min |
| **Flow-rate:** | 0.8 ml/min |
| **Detection:** | UV, 220 nm |
| **Injection volume:** | 5 µl |
| **Column temperature:** | 20°C |
| **Diluent:** | 50% acetonitrile |

### Sample preparation:

Accurately weigh about 6 mg of sample into 20 ml volumetric flask, dissolve in 2.5 ml of DMSO and dilute to volume with diluent.

### Calculation:

Use area per cent method. Do not integrate solvent peaks.

### Water content

Water content may be determined by Karl Fischer method. In particular, water content in the sample can be determined according to European Pharmacopoeia 6.0, section 2.5.12.

The following examples illustrate the invention and are not intended to restrict the scope of the invention in anyway.

### Examples

### Example 1

### Synthesis of a ethyl 1-(4-methoxyphenyl)-6-(4-nitrophenyl)-7-oxo-4,5,6,7-tetrahydro-1H-pyrazolo[3,4-c]pyridine-3-carboxylate (I)

In a 10 L reactor is charged 3.2 L of toluene, 461.5g of 3-chloro-1-(4-nitrophenyl)-5,6-dihydropyridin-2(1H)-one, 515.7 g of ethyl (Z)-2-chloro-2-(2-(4-methoxyphenyl)hydrazono)acetate. Triethylamine 611 mL is added dropwise at 75°C, after the addition reaction mixture is stirred at 75°C for 5 h. After the reaction is completed, reaction mixture is cooled to 5°C. The product is isolated by filtration in filter dryer and slurried in 4.6L of water at room temperature. Yellow solid is obtained (715 g yield 90%). The product is used for the next step without further purification.

### Example 2

### Synthesis of ethyl 6-(4-aminophenyl)-1-(4-methoxyphenyl)-7-oxo-4,5,6,7-tetrahydro-1H-pyrazolo[3,4-c]pyridine-3-carboxylate (II)

In a 10 L reactor is charged 762.4 g of ethyl 1-(4-methoxyphenyl)-6-(4-nitrophenyl)-7-oxo-4,5,6,7-tetrahydro-1H-pyrazolo[3,4-c]pyridine-3-carboxylate (I), 4500 mL of N, N-Dimethylformamide. Reactor is inertised and a catalyst 5% Pd/C 50.89 g is added. Reaction mixture is stirred under hydrogen at 45°C for 5 h. After the reaction is completed, catalyst is removed by filtration. The product was precipitated from filtrate by dropwise addition to water at 50°C. Suspension is stirred at 50°C for 1 h and then cooled to 5°C. Product is isolated by filtration in filter dryer, DMF is removed by slurring in water. Yellow crystalline solid is obtained (841 g, LOD=20%). The product is used for the next step without further purification and drying.

### Example 3

### Synthesis of ethyl 6-(4-(5-chloropentanamido)phenyl)-1-(4-methoxyphenyl)-7-oxo-4,5,6,7-tetrahydro-1H-pyrazolo[3,4-c]pyridine-3-carboxylate (III)

In a 10 L reactor is charged 775 g of wet ethyl 6-(4-aminophenyl)-1-(4-methoxyphenyl)-7-oxo-4,5,6,7-tetrahydro-1H-pyrazolo[3,4-c]pyridine-3-carboxylate (II), 2,9 L methylene chloride. Reaction mixture is stirred at RT until a solid is dissolved. Reaction mixture is cooled to 15°C, and 212 mL of N,N,N-triethylamine is added dropwise. After the addition 216 mL of 5-chlorovaleroyl chloride is added dropwise. Reaction mixture is stirred for 1 h. After completion temperature is raised to 22°C and the water 3.1 L is added. Phases are separated, the product is precipitated from organic phase by addition of 5 L ethyl acetate. Suspension is stirred at 55°C for 1 h and cooled to -20°C. Product is isolated by filtration. Beige solid is obtained (741 g, yield: 93%). The product is used for the next step without further purification.

### Example 4

### Synthesis of ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV)

In a 100 mL reactor is charged 15 g ethyl 6-(4-(5-chloropentanamido)phenyl)-1-(4-methoxyphenyl)-7-oxo-4,5,6,7-tetrahydro-1H-pyrazolo[3,4-c]pyridine-3-carboxylate (III), 50 mL of acetonitrile. Potassium carbonate (3.95 g) and N,N,N,N-tetrabutylammonium bromide (307.02 mg) are added to reaction mixture. Suspension is stirred under nitrogen atmosphere at 75°C for 20 h. After a reaction is completed, salts are removed by hot filtration. Product is precipitated from filtrate by solvent exchange with ethyl acetate at 65°C. Product is isolated by filtration. Beige solid is obtained (12.7 g, yield 91%). The product is used for the next step without further purification.

Solvent exchange in Example 4 is performed by evaporation of acetonitrile until LOD of 60-65% is achieved. Ethyl acetate is than added into reaction mixture (8 vol. eq). Evaporation is continued until LOD (loss of drying) of 80-85% is achieved. 4 vol. eq of ethyl acetate is added and evaporation is repeated until LOD of 85-80% is obtained. At the end 4 vol. eq of ethyl acetate is added.

### Example 5

### Comparative experiments concerning the synthesis of ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV)

For highlighting unexpected and surprising advantageous technical effects of using potassium carbonate in the cyclization step (ii), as identified above, comparative experiments with several other bases were carried out:
When carrying out in a comparative experiment the reaction with trimethylamine, only 0.25% of product was obtained. When carrying out in a comparative experiment the reaction with potassium tert butoxide, 70% of product was obtained, however (at least) two impurities were obtained in significant amounts (in HPLC area%: 11%).

When carrying out in a comparative experiment the reaction with potassium carbonate in absence of phase transfer catalyst, after 24 hours at 75°C still 23% of unreacted starting material was present. When we added phase transfer catalyst TBAB reaction ended in 17 hours. Product was obtained in 98% purity.

Use of potassium carbonate with TBAB phase transfer catalyst significantly reduces industrial costs of API, for approximately 30%. It also reduces time of reaction, it is easy handled, and we obtain product with higher purity.

### Example 6

### Synthesis of 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxamide (V)

In a 10 L reactor is charged 350 g of ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV), 6 L of Methanol and 1.3 kg ammonia are added. Reaction mixture is stirred at 95°C for 10 h under pressure (900-1200 kPa) and then cooled to 5°C. Suspension is stirred at that temperature for 1 h. Product is isolated by filtration. With this process of apixaban preparation we obtained a product with high purity, all impurities are HPLC area <0.10% and pure N-1 polymorphic form.

### Example 7

### Additional experiments concerning the preparation of apixaban by reacting compound of formula (IV) with ammonia in methanol

Additional experiments concerning the preparation of apixaban by reacting compound of formula (IV) with ammonia in methanol were carried out, wherein several specific parameters were varied:

### a) Varying the reaction temperature

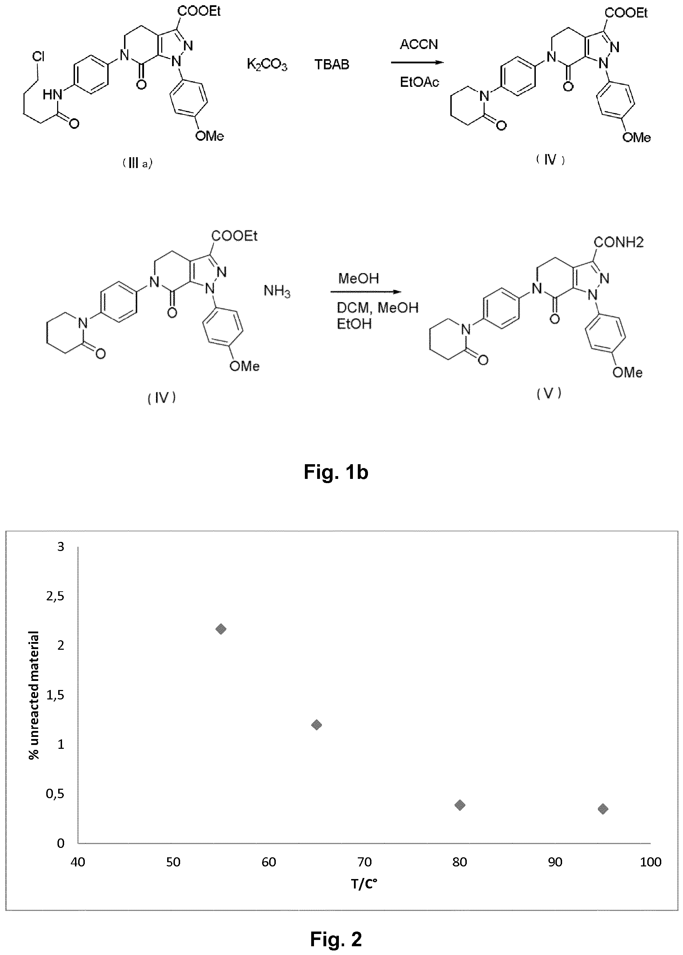

As shown in Figure 2, by applying a reaction temperature of 80°C or more unexpectedly the quantity of unreacted material can be minimized:

When the reaction was performed at 80°C or 95°C quantity of unreacted intermediate merely was 0.3% - 0.4%.

**Table 3**

| Experiment No. | Reaction time/h | Temperature/°C | %Unreacted material |
|---|---|---|---|
| 1 | 16 h | 55 | 2.17 |
| 2 | 16h | 65 | 1.20 |
| 3 | 16h | 80 | 0.39 |
| 4 | 16h | 95 | 0.35 |

Figure 2 depicts the relationship between unreacted material and temperature.

### b) Preparing apixaban by aminolysis with aqueous ammonia

Synthesizing apixaban by aminolysis with aqueous ammonia can be also carried out. Reacting compound of formula (IV) with ammonia in methanol shows the advantage of contributing to avoiding or reducing the content of acid impurity AXN461.

### c) Varying the concentration of starting material (intermediate IV)

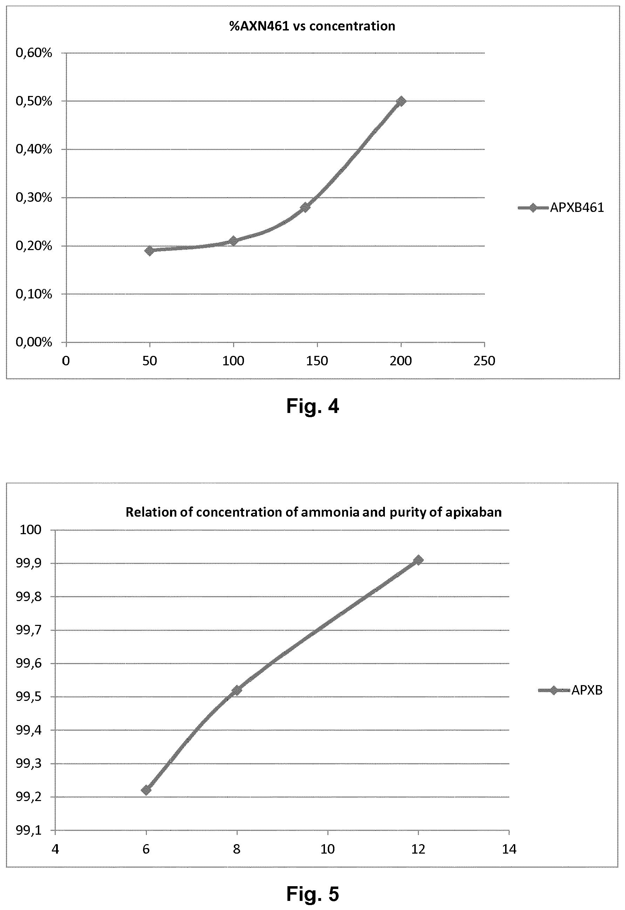

Presence of a low concentration of starting material (intermediate IV) in the reaction mixture surprisingly contributes to a reduction of the content of acid impurity AXN461 (also referred to herein as APXB461), as may be seen from the following Table and Figure 4.

**Table 4**

| **Exp.** | **Reaction time. (h)** | **Temp. (°C)** | **Dilution (mg/mL)** | **solvent** | **RRt/assignment** |
|---|---|---|---|---|---|
| | | | | | **Impurity AXN461** |
| **A_1** | precipitate10h | 95 | 100 | 12M (mol/L) NH₃/MeOH, **KF = 0.05%** | 0.21 |
| **A_2** | precipitate 10h | 95 | 67 | 12M (mol/L) NH₃/MeOH, **KF = 0.05%** | 0.27 |
| **A_3** | precipitate 10h, cooling to 17°C @ 0.2K/min | 95 | 67 | 12M (mol/L) NH₃/MeOH, **KF = 0.05%** | 0.06 |
| **A_4** | precipitate 10h | 95 | 100 | 12M (mol/L) NH₃/MeOH, **KF = 0.05%** | 0.07 |
| **A_5** | precipitate | 95 | 143 | 8M (mol/L) NH₃/MeOH, **KF = 0.05%** | 0.28 |
| **A_6** | precipitate | 95 | 200 | 8M (mol/L) NH₃/MeOH, **KF = 0.05%** | 0.50 |
| **A_7** | precipitate 16h | 95 | 50 | 8M (mol/L) NH₃/MeOH, **KF = 0.05%** | 0.19 |

| | | | | | |
|---|---|---|---|---|---|
| KF- means a content of water as determined by Karl Fisher. | | | | | |

Impurities AXN 461 (the terms AXN 461 and APXB461 are used interchangeably herein) and AXN475 referred to herein are:

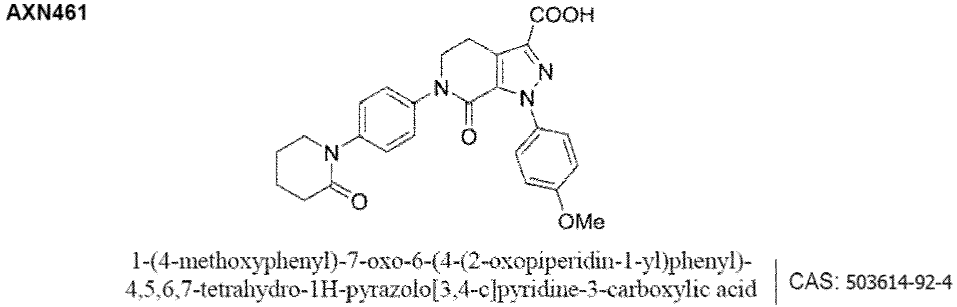

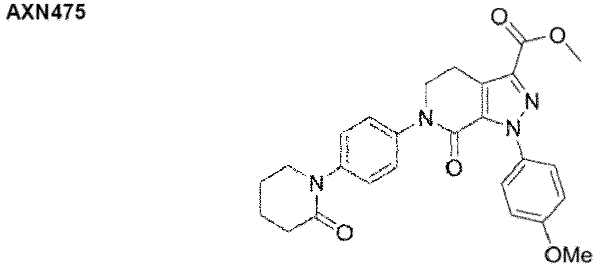

### methyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (CAS: 1074365-84-6)

From the following Table 5, it is evident that unexpectedly lower concentrations of starting material (dilution mg/mL) in the reaction mixture may contribute to reducing the content of acid impurity AXN461. Lower concentrations of starting material (compound (IV)) can therefore contribute to providing a product with particularly advantageous purity. The relationship between concentration and impurity formation is shown in Figure 4.

### d) Varying the concentration of ammonia

The concentration of ammonia (and consequently of the pressure used during reaction) surprisingly influences the reaction conversion. Carrying out the reaction with lower concentration of methanolic ammonia results in lower rate of conversion, and consequently may increase reaction time and formation of acid impurity AXN461 and impurity APXB475.

**Table 5.**

| **experiment** | **React.time. (h)** | **(°C)** | **Dilution (mg/mL)** | **solvent** | **RRt/assignment** | | | |
|---|---|---|---|---|---|---|---|---|
| | | | | | **AXN461** | **APXB** | **AXN475** | **APXB-8** |
| **B_1** | Precipitate10h, cooling to 10°C @ 0.2K/min | 95 | 50 | 12M (mol/L) NH₃/MeOH, **KF = 0.05%** | 0.04 | 99.91 | 0.05 | / |
| **B-2** | Precipitate 10h, cooling to 17°C @ 0.2K/min | 95 | 50 | 8M (mol/L) NH₃/MeOH, **KF = 0.05%** | 0.07 | 99.52 | 0.11 | / |
| **B-3** | Precipitate 10h, cooling to 17°C @ 0.2K/min | 95 | 50 | 6M (mol/L) NH₃/MeOH, **KF = 0.05%** | 0.10 | 99.22 | 0.28 | / |
| **B-4** | Precipitate 24h, cooling to 10°C @ 0.15K/min | 95 | 50 | 4M (mol/L) NH₃/MeOH, **KF = 0.05%** | 0.04 | 99.33 | 0.48 | / |
| **B-5** | Precipitate 24h, cooling to 10°C @ 0.15K/min | 95 | 50 | 6M (mol/L) NH₃/MeOH, **KF = 0.05%** | 0.09 | 99.53 | 0.16 | / |

| | | | | | | | | |
|---|---|---|---|---|---|---|---|---|
| RRt: relative retention time; APXB: apixaban; AXN461, AXN475, APXB-8 being impurities; Dilution: Dilution of compound (IV) per 1 mL of reaction mixture. The terms APXB461 and AXN461 are used interchangeably herein. The terms APXB475 and AXN475 are used interchangeably herein. | | | | | | | | |

**Table 6.**

| **experiment** | **React.time. (h)** | **Temp. (°C)** | **Dilution (mg/mL)** | **solvent** | | **RRt/assignment** | | | |
|---|---|---|---|---|---|---|---|---|---|
| | | | | | | **AXN461** | **APXB** | **AXN475** | **APXB-8** |
| **C-1** | Precipitate 8h, cooling to 22°C @ 1K/min | 95 | 50 | 12M (mol/L) NH₃/MeOH, **KF = 0.05%** | | 0.09 | 99.71 | 0.11 | / |
| **C-2** | precipitate 9h, cooling to 20°C @ 0.5K/min | 95 | 50 | 12M (mol/L) NH₃/MeOH, **KF = 0.05%** | | 0.06 | 99.87 | 0.05 | / |
| **C-3** | Precipitate 9.5h, cooling to 20°C @ 0.3K/min | 95 | 50 | 12M (mol/L) NH₃/MeOH, **KF = 0.05%** | | 0.03 | 99.88 | 0.04 | / |
| **C-4** | Precipitate 10h, cooling to 5°C @ 0.2K/min | 95 | 50 | 12M (mol/L) NH₃/MeOH, **KF = 0.05%** | | 0.03 | 99.89 | 0.03 | / |
| **C-5** | Precipitate 10h, cooling to 10°C @ 0.2K/min | 95 | 50 | 12M (mol/L) NH₃/MeOH, **KF = 0.05%** | | 0.04 | 99.82 | 0.03 | / |

Figure 5 shows an unexpected influence of the concentration of ammonia in the reaction mixture on the formation of pure product apixaban (APXB).

In case a reaction mixture with a lower concentration of methanolic ammonia is used, the reaction time can be prolonged and the acid impurity formation can be increased.

### e) Varying the cooling rate

The Table supra shows that carrying out the reaction at 95°C, using anhydrous methanol, anhydrous or essentially anhydrous conditions (water content of the reaction mixture of 0.05% or below, applying a concentration of starting material (IV) in the reaction mixture of around 50 mg/mL, and a comparatively high concentration of ammonia (about 8 - 12M (mol/L) NH₃ in MeOH) provides particularly advantageous results. Furthermore, surprisingly also the cooling rate and the final temperature of isolation can advantageously contribute.

### Example 8

### Pilot batch experiment

In reactor after inertization is charged 7.3 kg of ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV), and 92 L of methanol. Suspension is cooled down to 5°C, 25 kg of ammonia is added in 1 h. Reaction mixture is stirred at 200 rpm, heated in 1 h to 95°C, stirred at that temperature for 15 h. After completion of reaction, reaction mixture is cooled down to 5°C in 6 h. Product is isolated by over pressure filtration. Wet cake is washed with 22 kg of cooled methanol. Apixaban of pure polymorphic form N-1 is formed, with average particle size 350 µm, milled by pico pin mill at 55000 rpm to 36µm.

### Example 9

### Recrystallization

In 10L reactor is charged 280 g of crude apixaban (V), 2 L of methylene chloride and 530 mL of methanol are added. Solution is stirred at 35°C for 30 min and filtered. Apixaban is precipitated with and addition of 5.6 L methanol at room temperature. Suspension is cooled in 2h to -20°C and stirred for 1 h. Product is isolated on filter dryer. We obtain pure N-1 polymorphic form. To stabilize polymorphic form and to improve a drying of residual solvents we slurried a wet cake in 1.5 L ethanol at 55°C for 5 hours. Suspension is then cooled to room temperature and filtered. Wet cake is dried for 5 h at 50°C, 5 h at 70°c, and 5h at 80°C, p=50 mbar. Pure N-1 form is obtained, purity more than 99.90 % by HPLC, acid impurity not more than (nmt) 0.05%, any other nmt 0.10%. Pure polymorphic form N-1 is obtained, with average particle size 39µm, milled by pico pin mill with 55000 rpm to 13µm which is directly used in formulation studies.

### Example 10

### a) Recrystallization

In a cleaned and dried reactor is charged 4.8 kg of crude apixaban (V), 47.6 kg of methylene chloride and 7.1 kg of methanol are added. Solution is stirred at 33°C for 30 min with 80 rpm and filtered. Apixaban is precipitated with and addition of 75 kg methanol in 2 h at room temperature. Suspension is cooled in 2h to -15°C and stirred for 1 h. Product is isolated by overpressure filtration. We obtain pure N-1 polymorphic form. To stabilize polymorphic form and to improve a drying of residual solvents we slurried a wet cake in 22 kg of ethanol at 55°C for 5 hours with stirring 100 rpm. Suspension is than cooled to room temperature in 1.5 h and isolated by overpressure filtration. Wet cake is dried for 5 h at 50°C, 5 h at 70°C, and 5h at 80°C, p=50 mbar. Pure N-1 form is obtained, purity more than 99.90 % by HPLC, acid impurity nmt 0.05%, any other nmt 0.10%.Pure polymorphic form N-1 is obtained, with average particle size 116µm, milled by pico pin mill with 55000 rpm to 18µm which is directly used in formulation studies.

### b) Recrystallization

A further way to prepare pure polymorph N-1 is by directly over pressure filtration of reaction mixture, at temperature higher than 70°C and pressure 600-1000 kPa and precipitation of product by controlled cooling. With that procedure we increase a yield for more than 10%, we obtain pure product all impurities are under 0.10% HPLC area. We decrease a quantity of solvent in the last step.

### Example 11

### Synthesis of 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxamide (V)

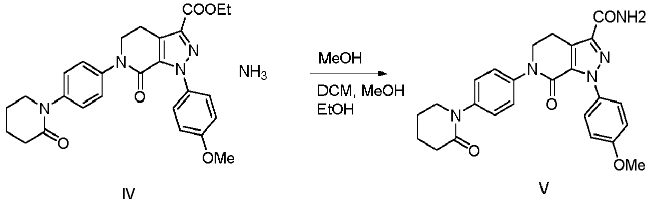

In a 10 L reactor is charged 350 g of ethyl 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxylate (IV), 6 L of Methanol and 1.3 kg ammonia are added. Reaction mixture is stirred at 95°C for 10 h under pressure and then cooled to 5°C. Suspension is stirred at that temperature for 1 h. Product is isolated by filtration . With this process of apixaban preparation, we obtained a product with high purity, all impurities are HPLC area <0.10% and pure N-1 polymorphic form.

### Example 12

### Milling of apixaban

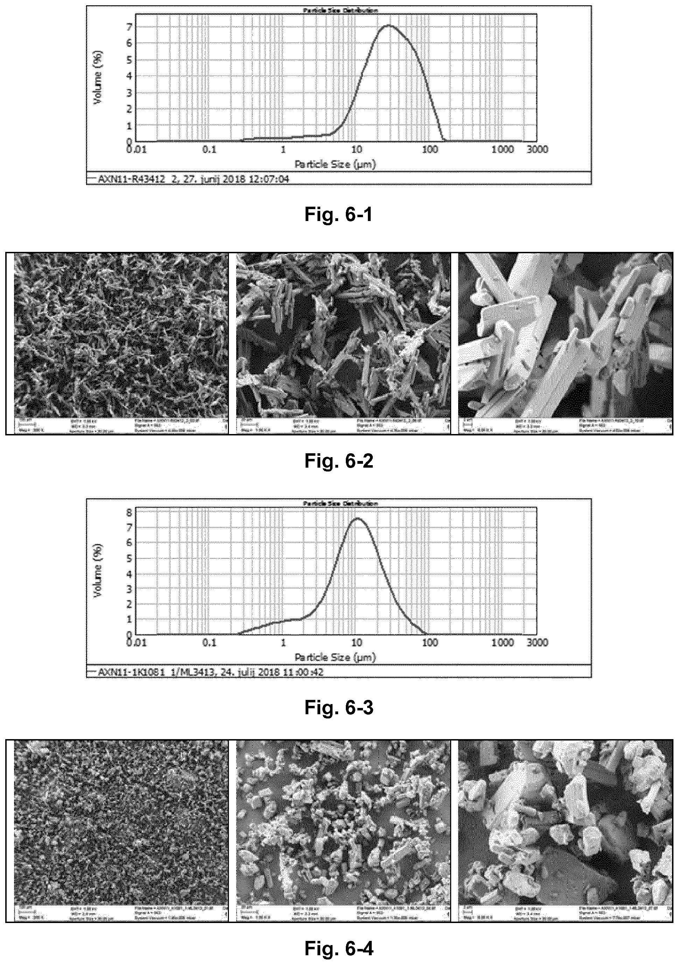

Material as obtained by process described in example 10 (R43412) characterized by PSD (Figure 6-1) and SEM (Figure 6-2) was milled on pin mill (Hosokawa Picomill). 100 g of material was fed in mill in app. 10 minutes with rotor speed 55000 RPM. Particles obtained by milling are characterized (AXN11-1K1081_1/ML3413) by PSD (Figure 6-3) and SEM image (Figure 6-4).

## Claims

1. A process for preparing apixaban of formula (V), 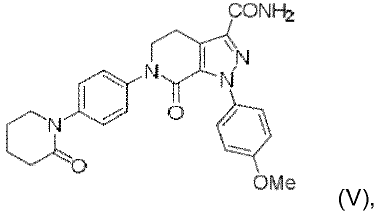 said process comprising:
(i) providing a compound of formula (III), 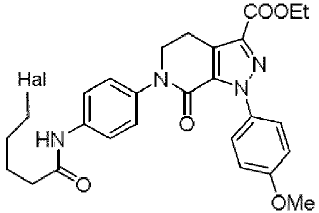 (III), wherein Hal is Br or Cl, preferably Cl;
(ii) cyclizing the compound of formula (III) in the presence of potassium carbonate, a first solvent, and a phase transfer catalyst, to obtain a compound of formula (IV) 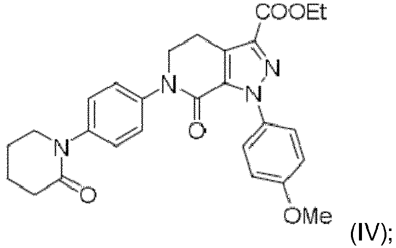
(iii) amidating the compound of formula (IV) to obtain apixaban of formula (V); and
(iv) optionally crystallizing apixaban of formula (V), especially optionally crystallizing apixaban polymorphic form N-1.

2. The process according to claim 1, **characterized in that** the phase transfer catalyst is or comprises a phase transfer catalyst selected from the group of N,N,N,N-tetraalkylammonium halogenides, N-benzyl-N,N,N-trialkylammonium halogenides and mixtures thereof,
preferably the phase transfer catalyst being or comprising a phase transfer catalyst selected from the group of N,N,N,N-tetraalkylammonium bromides and mixtures thereof, more preferably the phase transfer catalyst being or comprising N,N,N,N-tetrabutylammonium bromide.

3. The process according to any one of the preceding claims, **characterized in that** the first solvent is or comprises acetonitrile.

4. The process according to any one of the preceding claims, **characterized in that** step (iii) is or comprises reacting the compound of formula (IV) with NH₃ to obtain apixaban of formula (V), preferably at a temperature of 80°C or more,
optionally **characterized in that** step (iii) is or comprises reacting a mixture comprising the compound of formula (IV) and methanol with NH₃ to obtain apixaban of formula (V), at a temperature of 80°C or more.

5. The process according to any one of the preceding claims, wherein step (ii) comprises
(ii-1) cyclizing the compound of formula (III) in the presence of a potassium carbonate, a first solvent, and a phase transfer catalyst, to prepare a reaction mixture comprising the compound of formula (IV),
(ii-2) optionally subjecting the reaction mixture comprising the compound of formula (IV) to a removal of non-dissolved material from the reaction mixture, to obtain a solution comprising the compound of formula (IV),
in particular optionally subjecting the reaction mixture comprising the compound of formula (IV) to filtration to obtain a solution comprising the compound of formula (IV),
(ii-3) optionally removing the first solvent partially or completely from the solution comprising compound of formula (IV), and
(ii-4) adding a second solvent and allowing precipitation of compound of formula (IV), optionally the second solvent is or comprises ethyl acetate,
(ii-5) optionally isolating precipitate comprising or consisting of compound of formula (IV).

6. The process according to any one of the preceding claims, wherein step (i) of providing a compound of formula (III) comprises:
(A) contacting 3-chloro-1-(4-nitrophenyl)-5,6-dihydropyridin-2(1H)-one and ethyl 2-chloro-2-(2-(4-methoxyphenyl)hydrazono)acetate in the presence of a base to obtain a compound of formula (I) 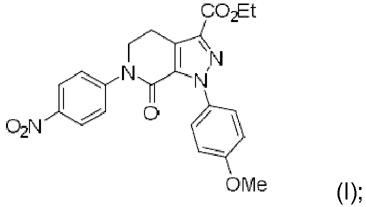
(B) reducing the compound of formula (I) in the presence of hydrogen to obtain a compound of formula (II) 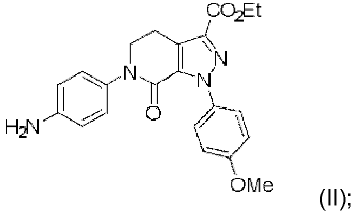
(C) contacting the compound of formula (II) with 5-halogenovaleroyl halogenide to obtain a compound of formula (III), 5-halogenovaleroyl halogenide being 5-chlorovaleroyl chloride or 5-bromovaleroyl chloride, 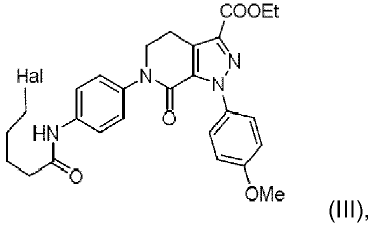 wherein Hal is Br or Cl.

7. The process according to claim 6, **characterized in that** step (A) comprises
(A-1) contacting 3-chloro-1-(4-nitrophenyl)-5,6-dihydropyridin-2(1H)-one and ethyl 2-chloro-2-(2-(4-methoxyphenyl)hydrazono)acetate in the presence of a base to obtain a reaction mixture comprising compound of formula (I),
(A-2) allowing precipitation of the compound of formula (I) from the reaction mixture to obtain a mixture comprising a precipitate comprising or consisting of compound of formula (I),
(A-3) isolating the precipitate comprising or consisting of compound of formula (I) from the mixture comprising a precipitate comprising or consisting of compound of formula (I),
(A-4) contacting the isolated precipitate comprising or consisting of compound of formula (I) with water to obtain a mixture comprising water and compound of formula (I),
(A-5) isolating compound of formula (I) from the mixture comprising water and compound of formula (I).

8. The process according to claim 6, **characterized in that** step (B) is reducing the compound of formula (I) in the presence of hydrogen and a hydrogenation catalyst to obtain a compound of formula (II), optionally the hydrogenation catalyst being a hydrogenation catalyst comprising one or more metals selected from platinum group (in particular Pd or Pt) and Ni, especially a hydrogenation catalyst comprising Pd, further especially Pd/C, Pd/Al₂O₃, and mixtures thereof, or **characterized in that** step (B) is reducing the compound of formula (I) in the presence of hydrogen, a hydrogenation catalyst, and a solvent to obtain a compound of formula (II), optionally the solvent being or comprising N,N-dimethylformamide (DMF).

9. The process according to any one of the claims 6 to 8, wherein step (B) comprises
(B-1) reducing the compound of formula (I) in the presence of hydrogen to prepare a reaction mixture comprising compound of formula (II),
(B-2) contacting the reaction mixture comprising compound of formula (II) with water to obtain a mixture comprising water and compound of formula (II),
(B-3) optionally agitating, preferably stirring, the mixture comprising water and compound of formula (II),
(B-4) isolating compound of formula (II) from the mixture comprising water and compound of formula (II).

10. The process according to any one of the preceding claims, **characterized in that** step (iii) is or comprises reacting the compound of formula (IV) with NH₃ in the presence of a solvent to obtain apixaban of formula (V), optionally said solvent being or comprising a solvent selected from the group of alkyl alcohols, further optionally said solvent being or comprising methanol.

11. Method for the preparation of crystalline apixaban of formula (V), especially apixaban polymorphic form N-1, said method comprising
(a) providing a mixture comprising apixaban of formula (V) and solvent at a first pressure and a first temperature, wherein the apixaban of formula (V) is at least partially present in not dissolved form at said first pressure and said first temperature in said mixture comprising apixaban of formula (V), and said solvent being methanol or a solvent mixture comprising or consisting of methanol and dichloromethane;
(b) subjecting the mixture comprising apixaban of formula (V) and solvent to a second pressure and a second temperature, said second pressure being a pressure at least 100 pascal higher than the first pressure, optionally said second pressure being a pressure at least 1 kilopascal higher than the first pressure (further optionally said second pressure being a pressure at least 100 kilopascal (kPa) higher than the first pressure), preferably to obtain a clear solution;
(c) optionally filtering the mixture obtained in step (b) to obtain a filtrate;
(d) allowing crystallization of crystalline apixaban, especially apixaban polymorphic form N-1, in the mixture obtained in step (b) at a third temperature below said second temperature and/or at a third pressure below said second pressure, or allowing crystallization of crystalline apixaban of formula (V) in the filtrate obtained in optional step (c) at a third temperature below said second temperature and/or at a third pressure below said second pressure;
(e) optionally isolating the crystalline apixaban of formula (V), especially apixaban polymorphic form N-1; and
(f) optionally drying the isolated crystalline apixaban of formula (V), especially apixaban polymorphic form N-1.

12. Method for the preparation of crystalline apixaban of formula (V) according to claim 11, **characterized in that** the crystalline apixaban is apixaban polymorphic form N-1.

13. Method for the preparation of crystalline apixaban of formula (V) according to any one of claims 11 to 12, **characterized in that** said second pressure is a pressure in the range from 102 kilopascal to1300 kilopascal, optionally from 200 kilopascal to 1000 kilopascal.

14. Method for the preparation of crystalline apixaban of formula (V) according to any one of claims 11 to 13, **characterized in that** the solvent is methanol, or a solvent mixture comprising or consisting of methanol and dichloromethane, optionally the solvent mixture having a weight ratio (methanol: dichloromethane) in the range of from 99 : 1 to 1 : 99, further optionally 70:30 to 90:10, preferably 80:20 to 90:10, or
**characterized in that** the method comprises a step prior to step (a), said step prior to step (a) comprising: preparing apixaban of formula (V) in the presence of methanol.

15. Method for the preparation of crystalline apixaban of formula (V), especially apixaban polymorphic form N-1, said method comprising
(a) providing a solution comprising apixaban of formula (V) dissolved in a solvent, said solvent being methanol or dichloromethane or a solvent mixture comprising or consisting of methanol and dichloromethane, optionally the solvent mixture having a weight ratio (methanol: dichloromethane) in the range of from 1 : 99 to 50: 50, further optionally in the range of from 10 : 90 to 30: 70;
(b) adding methanol to said solution comprising apixaban of formula (V) (optionally said solution having a temperature in the range of from 15 to 40 °C, further optionally in the range of from 25 to 35 °C), to precipitate crystalline apixaban of formula (V), wherein optionally methanol is added in such an amount to said solution comprising apixaban of formula (V) that the mixture obtained after the addition of methanol comprises apixaban of formula (V) and a solvent mixture comprising or consisting of methanol and dichloromethane, optionally the solvent mixture having a weight ratio (methanol:dichloromethane) in the range from 10:90 to 90:10, further optionally in the range of from 50:50 to 90:10, preferably 60:40 to 80:20;
(c) isolating the precipitated crystalline apixaban of formula (V), especially apixaban polymorphic form N-1 (in particular from the mixture obtained after the addition of methanol); and
(d) optionally slurrying the isolated crystalline apixaban of formula (V), especially apixaban polymorphic form N-1, in alcohol, optionally in alkyl alcohol, further optionally in C1-C5 alkyl alcohol, to obtain a slurry comprising the crystalline apixaban of formula (V);
(e) optionally isolating the crystalline apixaban of formula (V), especially isolating apixaban polymorphic form N-1, from the slurry comprising the crystalline apixaban of formula (V), especially from the slurry comprising apixaban polymorphic form N-1;
(f) optionally drying the isolated crystalline apixaban, especially drying the isolated apixaban polymorphic form N-1.
